(19) Europäisches Patentamt
European Patent Office
Office européen des brevets

(11) EP 4 289 829 A1

(12) EUROPEAN PATENT APPLICATION

(43) Date of publication:
**13.12.2023 Bulletin 2023/50**

(21) Application number: **22178275.8**

(22) Date of filing: **10.06.2022**

(51) International Patent Classification (IPC):
***C07D 273/04*** *(2006.01)*

(52) Cooperative Patent Classification (CPC):
**C07D 273/04**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Bayer Aktiengesellschaft**
**51373 Leverkusen (DE)**

(72) Inventor: **The designation of the inventor has not yet been filed**

(74) Representative: **BIP Patents**
**c/o Bayer Intellectual Property GmbH**
**Alfred-Nobel-Straße 50**
**40789 Monheim am Rhein (DE)**

(54) **CRYSTALLINE FORMS OF (6S)-5-[4'-FLUORO-2-(TRIFLUOROMETHYL)BIPHENYL-4-YL]-6-METHYL-3,6-DIHYDRO-2H-1,3,4-OXADIAZIN-2-ONE**

(57) This present invention relates to a crystalline form of (6S)-5-[4'-Fluoro-2-(trifluoromethyl)biphenyl-4-yl]-6-methyl-3,6-dihydro-2H-1,3,4-oxadiazin-2-one which is the modification C, to processes for its preparation, to pharmaceutical compositions comprising it and to its use in the treatment of disorders.

EP 4 289 829 A1

## Description

This present invention relates to the crystalline form of (6S)-5-[4'-Fluoro-2-(trifluoromethyl)biphenyl-4-yl]-6-methyl-3,6-dihydro-2H-1,3,4-oxadiazin-2-one which is the modification C, to processes for its preparation, to pharmaceutical compositions comprising it and to its use in the control of disorders. (6S)-5-[4'-Fluoro-2-(trifluoromethyl)biphenyl-4-yl]-6-methyl-3,6-dihydro-2H-1,3,4-oxa-diazin-2-one corresponds to the compound of formula (I):

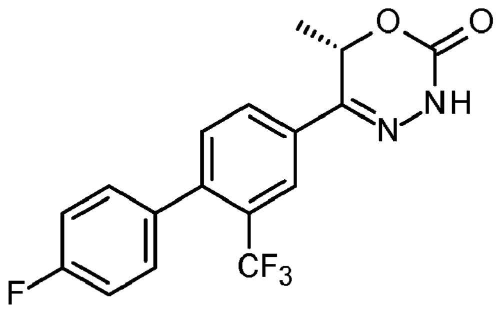

(I).

## BACKGROUND

This compound is first disclosed in WO2019/025562, example 135. No information about its solid state nor a hint that the compound even existed in different modifications was provided therein.

Its crystalline form A and its crystalline form B are published.

The following crystalline forms of the compound of formula (I) have been identified which are modification A, modification B, and the pseudopolymorphs which are the formamide solvate and a sulfolane solvate. Both could be obtained by following the synthesis as described in WO2019/025562 either as single modification or as a mixture thereof. In this context the terms "crystalline forms", "modifications", and "polymorphs" have the same meaning. In addition the amorphous form exists. All together - the modifications, the pseudopolymorphs and the amorphous form - are different solid forms of the compound of formula (I).

## SUMMARY

Now in addition modification C of the compound of the formula (I) has been identified which seems to be the thermodynamically stable form between 0 °C and 80 °C. It was obtained for the first time by having used the already published synthesis, formerly leading to modification A and/or B having by incident stored the product for quite some time at room temperature and having recrystallized the product thereafter. Surprisingly modification C of the compound of formula (I) was identified and it was discovered that modification C shows beneficial properties over the other known solid forms of the compound of formula (I) with regard to its habitus, particle size distribution, its bulk density, storage volume and storage stability.

Modification C is therefore suitable and preferred over the other solid forms of the compound of formula I for use in the pharmaceutical field, in particular better suitable for preparation of pharmaceutical compositions and for the pharmaceutical preparations themselves.

In particular the use of modification C of the compound of the formula (I) ensures that an undesired conversion into another form of the compound of formula (I) and an associated change in the properties as described above is prevented. This increases the safety and quality of compositions, preparations and formulations comprising the compound of the formula (I) and the risk to the patient is reduced.

A pharmaceutical composition according to the present invention comprises modification C of the compound of formula (I) and optionally further pharmaceutically acceptable excipients.

## BRIEF DESCRIPTION OF THE DRAWINGS

Figure 1: X-Ray powder diffractogram of modification C, as obtained in example 1.5

Figure 2: X-Ray powder diffractogram of modification C, as obtained in example 1.5, second batch which is one of the other five batches, which were combined for micronization.

Figure 3: X-Ray powder diffractogram of modification A, as obtained in example 1.6

Figure 4: X-Ray powder diffractogram of modification B, the product obtained in example 1.7

Figure 5: X-Ray powder diffractogram of modification B, the product obtained in example 1.8, 1)

Figure 6: X-Ray powder diffractogram of modification C, the product obtained in example 1.8, 2)

Figure 7: X-Ray powder diffractogram of modification A, the product as obtained in example 2.1, table in example 3.1

Figure 8: X-Ray powder diffractogram of modification B, the product as obtained in example 2.2, table in example 3.1

Figure 9: X-Ray powder diffractogram of modification C, the product as obtained in example 2.3, table in example 3.1

Figure 10: IR-Spectrum of modification A as obtained in example 2.1, table in example 3.2

Figure 11: IR-Spectrum of modification B as obtained in example 2.2, table in example 3.2

Figure 12: IR-Spectrum of modification C as obtained in example 2.3, table in example 3.2

Figure 13: Raman-Spectrum of modification A as obtained in example 2.1, table in example 3.3

Figure 14: Raman-Spectrum of modification B as obtained in example 2.2, table in example 3.3

Figure 15: Raman-Spectrum of modification C as obtained in example 2.3, table in example 3.3

Figure 16: DSC- and TGA-thermogram of modification A as obtained in example 3.4 showing Heat Flow Endo up [mW] vs Temperature [°C] as the peak curve and the red line curve reflects Weight % [%] vs temperature

Figure 17: DSC- and TGA-thermogram of modification B as obtained in example 3.4 showing Heat Flow Endo up [mW] vs Temperature [°C] as the peak curve and the red line curve reflects Weight % [%] vs temperature

Figure 18: DSC- and TGA-thermogram of modification C as obtained in example 3.4 showing Heat Flow Endo up [mW] vs Temperature [°C] as the peak curve and the red line curve reflects Weight % [%] vs temperature

Figure 19A: Schulze Ring Shear Tester device

Figure 19B: Shear Cell XS-SV3 belonging to the Schulze Ring Shear Tester device of figure 19A

Figure 20: Result of the Schulze Ring Shear Test for probe 1 (modification A), obtained in example 1.6 and probe 2 (modification C) obtained in example 1.5.

Figure 21: Illustration of the flow factors of modification A and modification C calculated on basis of the results of the Schulze Ring Shear Test according to example 3.

Figure 22: Result of the determination of bulk density during shear test, see example 4. Bulk density of sample 2 (Probe 2) is significantly higher than bulk density of sample 1 (Probe 1).

Figure 23: Comparison of particle size distribution of modification A (sample 1) and C (sample 2), see example 5, each sample without ("ohne") and with 300s of ultrasound treatment

Figure 24A: Particle size distribution of modification A without- and under ultrasound treatment, see example 5, "Fraunhofer Auswertung" means Fraunhofer Analysis Method

Figure 24B: Particle size distribution of modification C without- and under ultrasound treatment, see example 5, "Fraunhofer Auswertung" means Fraunhofer Analysis Method

## DETAILED DESCRIPTION

A preferred embodiment of the present invention is a pharmaceutical composition comprising mainly modification C of the compound of formula (I) and no significant fractions of another form of the compound of formula (I) and optionally further pharmaceutically acceptable excipients. More preferably the pharmaceutical composition contains more than 85 percent by weight, more preferably more than 90 percent by weight, most preferably more than 95 percent by weight, of the modification C of the compound of formula (I) related to the total amount of all forms of the compound of formula (I) present in the composition. A significant fraction of another modification than modification C is considered 15 % per weight or more.

The different forms of the compound of formula (I) can be distinguished by X-ray powder diffraction, IR-, Raman spectroscopy and. differential scanning calorimetry (DSC),

### X-Ray powder diffractograms

The modification C of the compound of formula (I) can be characterized unambiguously by a X-Ray powder diffractogram (at 25°C and with Cu-K alpha 1 as radiation source) which displays at least the following reflections: 21,1; 15,1; 22,6; preferably at least the following reflections: 21,1; 15,1; 22,6; 18,8; 26,8; more preferably at least the following reflections: 21,1; 15,1; 22,6; 18,8; 26,8; 22,6; 18,9; most preferably at least the following reflections: 21,1; 15,1; 22,6; 18,8; 26,8; 22,6; 18,9; 29,8; 11,9; 31,6; each quoted as 2Θ value ± 0.2°. The compound of formula (I) in the modification C can also be characterized unambiguously by the X-Ray powder diffractogram (at 25°C and with Cu-K alpha 1 as radiation source) as shown in Figure 1.

The modification B of the compound of formula (I) can be characterized unambiguously by a X-Ray powder diffractogram (at 25°C and with Cu-K alpha 1 as radiation source) which displays at least the following reflections: 20,5; 20,8; 16,9; preferably at least the following reflections: 20,5; 20,8; 16,9; 21,5; 23,7; more preferably at least the following reflections: 20,5; 20,8; 16,9; 21,5; 23,7; 14,4; 26,8; most preferably at least the following reflections: 20,5; 20,8; 16,9; 21,5; 23,7; 14,4; 26,8; 27,2; 16,7; 12,4; each quoted as 2Θ value ± 0.2°. The compound of formula (I) in the modification B can also be characterized unambiguously by the X-Ray powder diffractogram (at 25°C and with Cu-K alpha 1 as radiation source) as shown in Figure 2.

The modification A of the compound of formula (I) can be characterized unambiguously by a X-Ray powder diffractogram (at 25°C and with Cu-K alpha 1 as radiation source) which displays at least the following reflections: 20,7; 20,5; 12,3 preferably at least the following reflections: 20,7; 20,5; 12,3; 17,0; 23,9; more preferably at least the following reflections: 20,7; 20,5; 12,3; 17,0; 23,9; 21,0; 27,4; most preferably at least the following reflections: 20,7; 20,5; 12,3; 17,0; 23,9; 21,0; 27,4; 13,9; 21,5; 15,2; each quoted as 2Θ value ± 0.2°. The compound of formula (I) in the modification A can also be characterized unambiguously by the X-Ray powder diffractogram (at 25°C and with Cu-K alpha 1 as radiation source) as shown in Figure 3.

### IR spectra

The modification C of the compound of formula (I) can be characterized unambiguously by IR spectroscopy using an ATR (attenuated total reflection) unit without sample preparation which displays at least the following reflections: 1123, 837, 1285 [cm-1], preferably at least the following reflections: 1123, 837, 1285, 678, 1164 [cm-1], more preferably at least the following reflections: 1123, 837, 1285, 678, 1164, 1730, 1049 [cm-1], most preferably at least the following reflections: 1123, 837, 1285, 678, 1164, 1730, 1049, 1175, 659 [cm-1], each quoted as band maxima ± 2 cm-1. The compound of formula (I) in the modification C can also be characterized unambiguously by the IR spectrum (using an ATR unit without sample as shown in Figure 5.

The modification B of the compound of formula (I) can be characterized unambiguously by IR spectroscopy (using an ATR unit without sample preparation) which displays at least the following reflections: 833, 1059, 1128 [cm-1], preferably at least the following reflections: 833, 1059, 1128, 1274, 661 [cm-1], more preferably at least the following reflections: 833, 1059, 1128, 1274, 661, 1732, 656 [cm-1], most preferably at least the following reflections: 833, 1059, 1128, 1274, 661, 1732, 656, 591, 907, 724 [cm-1], each quoted as band maxima ± 2 cm-1. The compound of formula (I) in the modification B can also be characterized unambiguously by the IR spectrum (using an ATR unit without sample preparation) as shown in Figure 6.

The modification A of the compound of formula (I) can be characterized unambiguously by IR spectroscopy (at using an ATR unit without sample preparation) which displays at least the following reflections: 1123, 1063, 1275 [cm-1], preferably at least the following reflections: 1123, 1063, 1275, 1732, 834 [cm-1], more preferably at least the following reflections: 1123, 1063, 1275, 1732, 834, 829, 656 [cm-1], most preferably at least the following reflections: 1123, 1063, 1275, 1732, 834, 829, 656, 908, 1175, 717 [cm-1], each quoted as band maxima ± 2 cm-1. The compound of formula (I) in the modification A can also be characterized unambiguously by the IR spectrum (using an ATR unit without sample

preparation) as shown in Figure 7.

**Raman spectra**

The modification C of the compound of formula (I) can be characterized unambiguously by Raman spectroscopy (at 25°C using a 1064 nm NdYAG Laser) which displays at least the following reflections: 1606, 1617, 141 [cm-1], preferably at least the following reflections: 1606, 1617, 141, 92, 1272 [cm-1], more preferably at least the following reflections: 1606, 1617, 141, 92, 1272, 120, 1328 [cm-1], most preferably at least the following reflections: 1606, 1617, 141, 92, 1272, 120, 1328, 812, 1095, 1298 [cm-1], each quoted as band maxima ± 2 cm-1. The compound of formula (I) in the modification C can also be characterized unambiguously by the Raman spectrum (at 25°C using a 1064 nm NdYAG Laser) as shown in Figure 8.

The modification B of the compound of formula (I) can be characterized unambiguously by Raman spectroscopy (at 25°C using a 1064 nm NdYAG Laser) which displays at least the following reflections: 1610, 1252, 815 [cm-1], preferably at least the following reflections: 1610, 1252, 815, 1070, 1449 [cm-1], more preferably at least the following reflections: 1610, 1252, 815, 1070, 1449, 1339, 1464 [cm-1], most preferably at least the following reflections: 1610, 1252, 815, 1070, 1449, 1339, 1464, 1300, 1432, 1632 [cm-1], each quoted as band maxima ± 2 cm-1. The compound of formula (I) in the modification B can also be characterized unambiguously by the Raman spectrum (at 25°C using a 1064 nm NdYAG Laser) as shown in Figure 9.

The modification A of the compound of formula (I) can be characterized unambiguously by Raman spectroscopy (at 25°C using a 1064 nm NdYAG Laser) which displays at least the following reflections: 1612, 1252, 815 [cm-1], preferably at least the following reflections: 1612, 1252, 815, 1070, 1433 [cm-1], more preferably at least the following reflections: 1612, 1252, 815, 1070, 1433, 1447, 1463 [cm-1], most preferably at least the following reflections: 1612, 1252, 815, 1070, 1433, 1447, 1463, 1286, 1107, 139 [cm-1], each quoted as band maxima ± 2 cm-1. The compound of formula (I) in the modification A can also be characterized unambiguously by the Raman spectrum (at 25°C using a 1064 nm NdYAG Laser) as shown in Figure 10.

Process for preparing:

The compound of formula (I) can be prepared according to the methods disclosed in WO2019/025562 or following the route as described in Schemes 1a-c below:

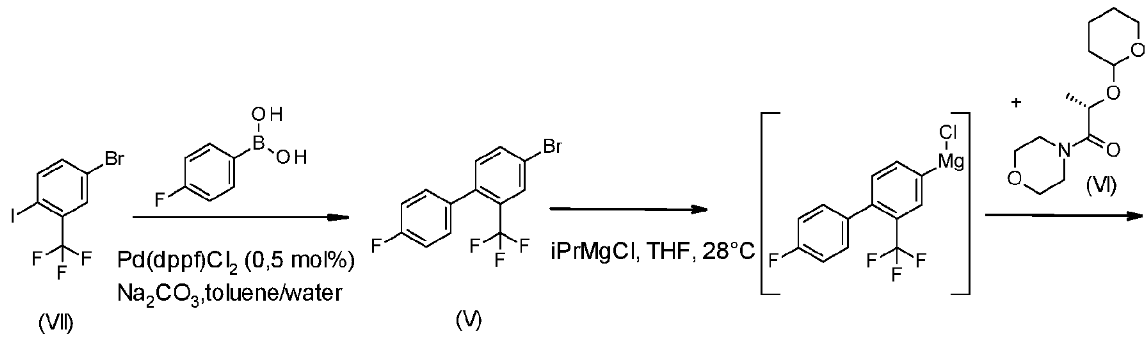


Scheme (Ia)

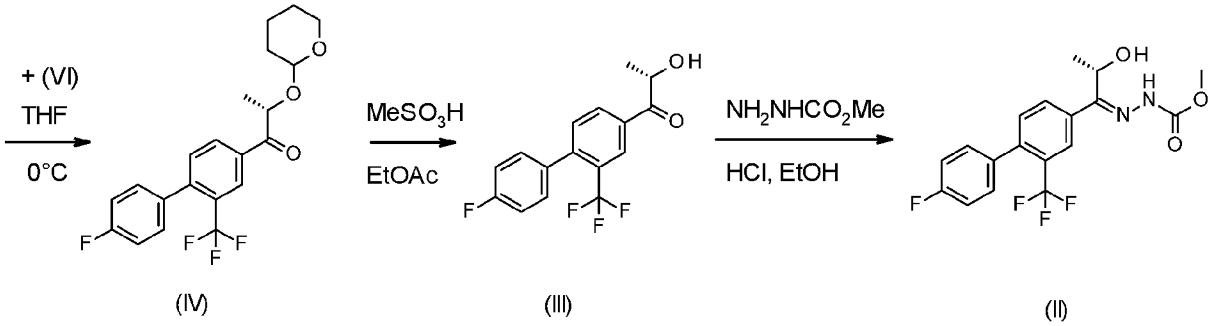


Scheme (Ib)

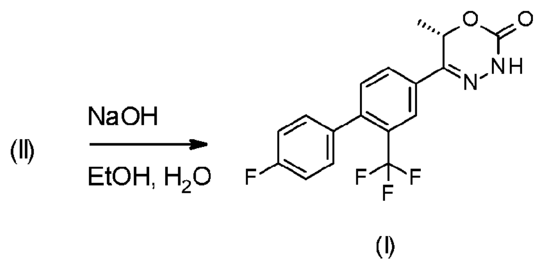


Scheme (Ic)

Schemes (Ia-Ic): *Convergent synthesis of the compound of formula (I)*

This synthesis is reflected in the experimental section.

**Definitions**

The terms "sample" and "probe" are used synonymously herein.

The terms "crystalline forms", "forms" "modifications", and "polymorphs" have the same meaning.

"Another form" of a compound of formula (I) in a composition comprising mainly modification C can be any other modification than C, especially modification A or modification B. And if the plural term "other forms" is used it can also comprise mixtures of any of the modifications , especially mixtures of modification A and B besides C.

A significant fraction of another modification than modification C in a sample is considered 15 % per weight or more.

## Method for treatment

The crystalline forms of the compound of formula (I), preferably modification C according to the invention may have useful pharmacological properties and may be employed for the prevention and treatment of disorders in humans and animals.

The crystalline form C of the compound of formula (I) according to the invention can be used for the treatment of hyperproliferative diseases, especially cancer, more especially where the cancer disease occurs in the following organs: anus, the brain, the breast, the bones, the central and peripheral nervous system, the colon, the eye, the kidney, the endocrine glands (e.g., thyroid and adrenal cortex), the endometrium, the esophagus, the gastrointestinal tract (including gastrointestinal stromal tumors),the germ cells, the head and the neck, the kidney, the liver, the larynx and hypopharynx, the lung, the mesothelioma, the pancreas, the prostate, the rectum, the reproductive organs (e.g., cervix, ovary, prostate), the respiratory tract, the small intestine, the skin, the soft tissue, the stomach, the testis, the thyroid gland, the parathyroid gland, ureter, the urogenital tract, vagina and vulva and the connective tissue, including their metastases.

A preferred list of tumors for treatment of cancer are those occurring in the brain, female reproductive tract, especially ovary, and skin, and including sarcoma.

Examples of breast cancers include, but are not limited to, invasive ductal carcinoma, invasive lobular carcinoma, ductal carcinoma in situ, and lobular carcinoma in situ.

Examples of "brain cancers" include, but are not limited to, brain stem and hypophtalmic glioma, cerebellar and cerebral astrocytoma, medulloblastoma, ependymoma, as well as neuroectodermal and pineal tumour.

Tumors of the "central nervous system" include according to the WHO listing, but are not limited to, tumors of neuroepithelial tissue (e.g. astocytic tumors, gliomas), oligodentromial tumors, glioneuronal tumors and neuronal tumors, choroid plexus tumors, pineal tumors, cranial and paraspinal nerve tumors, melanocytic tumors, hematolymphoid tumors.

Tumours of the "digestive tract" include, but are not limited to, anal, colon, colorectal, oesophageal, gallbladder, gastric, pancreatic, rectal, small-intestine, and salivary gland cancers.

"Eye cancers" include, but are not limited to, intraocular melanoma and retinoblastoma.

"Head-and-neck cancers" include, but are not limited to, laryngeal, hypopharyngeal, nasopharyngeal, oropharyngeal cancer, lip and oral cavity cancer and squamous cell.

Examples of "liver cancers" include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Examples of cancers of the "respiratory tract" include, but are not limited to, small-cell and non-small-cell lung carcinoma, as well as bronchial adenoma and pleuropulmonary blastoma.

"Reproductive organs" include female- and male reproductive organs.

"Tumours of the female reproductive organs" include, but are not limited to, endometrial, cervical, ovarian, vaginal, and vulvar cancer, as well as sarcoma of the uterus. T"umours of the male reproductive organs" include, but are not limited to, prostate and testicular cancer.

"Skin cancers" include, but are not limited to, squamous cell carcinoma, Kaposi's sarcoma, malignant melanoma (melanoma), Merkel cell skin cancer, and non-melanoma skin cancer.

"Tumours of the urinary tract" include, but are not limited to, bladder, penile, kidney, renal pelvis, ureter, urethral and human papillary renal cancers.

"Lymphomas" include, but are not limited to, AIDS-related lymphoma, non-Hodgkin's lymphoma, cutaneous T-cell lymphoma, Burkitt lymphoma, Hodgkin's disease, and lymphoma of the central nervous system.

"Sarcomas" include, but are not limited to, sarcoma of the soft tissue, osteosarcoma, malignant fibrous histiocytoma, lymphosarcoma, and rhabdomyosarcoma.

"Leukemias" include, but are not limited to, acute myeloid leukemia (AML), acute lymphoblastic leukemia, chronic lymphocytic leukemia, chronic myelogenous leukemia, and hairy cell leukemia.

The crystalline form C of the compound of formula (I) according to the invention can furthermore be used for the treatment of a hyperproliferative disease, especially a cancer diseases, such as e.g. brain cancer, breast cancer, cervical cancer, head and neck cancer, leukemia, acute myeloid leukemia (AML), lung cancer (especially NCLC and SCLC), lymphoma, skin cancer (especially melanoma), esophagal carcinoma, ovarian cancer, pancreas cancer, prostate cancer and sarcoma.

In other embodiments the invention relates to the crystalline form C of the compound of formula (I) for use in the treatment and/or prophylaxis of diseases, especially hyperproliferative diseases, more especially cancer diseases.

In other embodiments the invention relates to the crystalline form C of the compound of formula (I) for use in the treatment and/or prophylaxis of diseases whereby the disease is a cancer disease which is selected from brain cancer, breast cancer, cervical cancer, head and neck cancer, leukemia, AML, lung cancer, lymphoma, skin cancer, esophagal carcinoma, ovarian cancer, pancreas cancer, prostate cancer and sarcoma.

Furthermore in other embodiments the invention relates to a pharmaceutical composition comprising modification C and optionally further comprising another form or other forms of the compound of formula (I), optionally with an amount of less than 15%, less than 10% or less than 5% for use in the treatment of a hyperproliferative disease.

In other embodiments the invention relates to a pharmaceutical composition comprising modification C and optionally further comprising another form or other forms of the compound of formula (I), optionally with an amount of less than 15%, less than 10% or less than 5%whereby the hyperproliferative disease is a cancer disease.

In further embodiments the invention relates to a pharmaceutical composition comprising modification C and optionally further comprising another form or other forms of the compound of formula (I), optionally with an amount of less than 15%, less than 10% or less than 5% whereby the cancer disease is selected from e.g. brain cancer, breast cancer, cervical cancer, head and neck cancer, leukemia, AML, lung cancer, lymphoma, skin cancer, esophagal carcinoma, ovarian cancer, pancreas cancer, prostate cancer and sarcoma.

In other embodiments the invention relates to the crystalline form C of the compound of formula (I) for use in the manufacture of a pharmaceutical composition for the treatment or prevention of a disease, especially a hyperproliferative disease, more especially a cancer disease, and yet even more especially a cancer disease selected from e.g. brain cancer, breast cancer, cervical cancer, head and neck cancer, leukemia, AML, lung cancer, lymphoma, skin cancer, esophagal carcinoma, ovarian cancer, pancreas cancer, prostate cancer and sarcoma.

Other embodiments of the invention include the crystalline form C of the compound of formula (I) for use in the manufacture of a stable pharmaceutical composition.

Further embodiments of the invention include a method of treating or preventing a hyperproliferative diseases, more especially a cancer disease, and yet even more especially a cancer disease selected from e.g. brain cancer, breast cancer, cervical cancer, head and neck cancer, leukemia, AML, lung cancer, lymphoma, skin cancer, esophagal carcinoma, ovarian cancer, pancreas cancer, prostate cancer and sarcoma in a mammal, comprising administering to a mammal in need thereof a therapeutically effective amount of modification C of the compound of formula (I).

In some embodiments, the present invention further relates to a method for the treatment and/or prophylaxis of diseases, in particular the aforementioned diseases, using an effective amount of at least one of the forms of the compound of formula (I) according to the invention, especially of modification C.

In some embodiments, the present invention further relates to a method for the treatment and/or prophylaxis of a hyperproliferative disease using an effective amount of at least one of the forms of the compound of formula (I) according to the invention, especially modification C, preferably the present invention further relates to a method for the treatment and/or prophylaxis of a cancer disease.

Combination

The crystalline forms of the compound of formula (I) according to the invention can be used alone or in combination with other active substances if necessary. The present invention further relates to medicinal products containing at least one of the forms of the compound of formula (I) according to the invention and one or more further active substances, in particular an anti-cancer agent, particularly for the treatment and/or prophylaxis of the aforementioned diseases. As suitable other active substances the following anti-cancer-agents can be mentioned: 131I-chTNT, abarelix, abemaciclib, abiraterone, acalabrutinib, aclarubicin, adalimumab, ado-trastuzumab emtansine, afatinib, aflibercept, aldesleukin, alectinib, alemtuzumab, alendronic acid, alitretinoin, altretamine, amifostine, aminoglutethimide, hexyl aminolevulinate, amrubicin, amsacrine, anastrozole, ancestim, anethole dithiolethione, anetumab ravtansine, angiotensin II, antithrombin III, apalutamide, aprepitant, arcitumomab, arglabin, arsenic trioxide, asparaginase, atezolizumab, avelumab, axicabtagene ciloleucel, axitinib, azacitidine, basiliximab, belotecan, bendamustine, besilesomab, belinostat, bevacizumab, bexarotene, bicalutamide, bisantrene, bleomycin, blinatumomab, bortezomib, bosutinib, buserelin, brentuximab vedotin, brigatinib, busulfan, cabazitaxel, cabozantinib, calcitonine, calcium folinate, calcium levofolinate, capecitabine, capromab, carbamazepine carboplatin, carboquone, carfilzomib, carmofur, carmustine, catumaxomab, celecoxib, celmoleukin, ceritinib, cetuximab, chlorambucil, chlormadinone, chlormethine, cidofovir, cinacalcet, cisplatin, cladribine, clodronic acid, clofarabine, cobimetinib, copanlisib , crisantaspase, crizotinib, cyclophosphamide, cyproterone, cytarabine, dacarbazine, dactinomycin, daratumumab, darbepoetin alfa, dabrafenib, dasatinib, daunorubicin, decitabine, degarelix, denileukin diftitox, denosumab, depreotide, deslorelin, dianhydrogalactitol, dexrazoxane, dibrospidium chloride, dianhydrogalactitol, diclofenac, dinutuximab, docetaxel, dolasetron, doxifluridine, doxorubicin, doxorubicin + estrone, dronabinol, durvalumab, eculizumab, edrecolomab, elliptinium acetate, elotuzumab, eltrombopag, enasidenib, endostatin, enocitabine, enzalutamide, epirubicin, epitiostanol, epoetin alfa, epoetin beta, epoetin zeta, eptaplatin, eribulin, erlotinib, esomeprazole, estradiol, estramustine, ethinylestradiol, etoposide, everolimus, exemestane, fadrozole, fentanyl, filgrastim, fluoxymesterone, floxuridine, fludarabine, fluorouracil, flutamide, folinic acid, formestane, fosaprepitant, fotemustine, fulvestrant, gadobutrol, gadoteridol, gadoteric acid meglumine, gadoversetamide, gadoxetic acid, gallium nitrate, ganirelix, gefitinib, gemcitabine, gemtuzumab, Glucarpidase, glutoxim, GM-CSF, goserelin, granisetron, granulocyte colony stimulating factor, histamine dihydrochloride, histrelin, hydroxycarbamide, 1-125 seeds, lansoprazole,

ibandronic acid, ibritumomab tiuxetan, ibrutinib, idarubicin, ifosfamide, imatinib, imiquimod, improsulfan, indisetron, incadronic acid, ingenol mebutate, inotuzumab ozogamicin, interferon alfa, interferon beta, interferon gamma, iobitridol, iobenguane (1231), iomeprol, ipilimumab, irinotecan, Itraconazole, ixabepilone, ixazomib, lanreotide, lansoprazole, lapatinib, lasocholine, lenalidomide, lenvatinib, lenograstim, lentinan, letrozole, leuprorelin, levamisole, levonorgestrel, levothyroxine sodium, lisuride, lobaplatin, lomustine, lonidamine, lutetium Lu 177 dotatate, masoprocol, medroxyprogesterone, megestrol, melarsoprol, melphalan, mepitiostane, mercaptopurine, mesna, methadone, methotrexate, methoxsalen, methylaminolevulinate, methylprednisolone, methyltestosterone, metirosine, midostaurin, mifamurtide, miltefosine, miriplatin, mitobronitol, mitoguazone, mitolactol, mitomycin, mitotane, mitoxantrone, mogamulizumab, molgramostim, mopidamol, morphine hydrochloride, morphine sulfate, mvasi, nabilone, nabiximols, nafarelin, naloxone + pentazocine, naltrexone, nartograstim, necitumumab, nedaplatin, nelarabine, neratinib, neridronic acid, netupitant/palonosetron, nivolumab, pentetreotide, nilotinib, nilutamide, nimorazole, nimotuzumab, nimustine, nintedanib, niraparib, nitracrine, nivolumab, obinutuzumab, octreotide, ofatumumab, olaparib, olaratumab, omacetaxine mepesuccinate, omeprazole, ondansetron, oprelvekin, orgotein, orilotimod, osimertinib, oxaliplatin, oxycodone, oxymetholone, ozogamicine, p53 gene therapy, paclitaxel, palbociclib, palifermin, palladium-103 seed, palonosetron, pamidronic acid, panitumumab, panobinostat, pantoprazole, pazopanib, pegaspargase, PEG-epoetin beta (methoxy PEG-epoetin beta), pembrolizumab, pegfilgrastim, peginterferon alfa-2b, pembrolizumab, pemetrexed, pentazocine, pentostatin, peplomycin, Perflubutane, perfosfamide, Pertuzumab, picibanil, pilocarpine, pirarubicin, pixantrone, plerixafor, plicamycin, poliglusam, polyestradiol phosphate, polyvinylpyrrolidone + sodium hyaluronate, polysaccharide-K, pomalidomide, ponatinib, porfimer sodium, pralatrexate, prednimustine, prednisone, procarbazine, procodazole, propranolol, quinagolide, rabeprazole, racotumomab, radium-223 chloride, radotinib, raloxifene, raltitrexed, ramosetron, ramucirumab, ranimustine, rasburicase, razoxane, refametinib, regorafenib, ribociclib, risedronic acid, rhenium-186 etidronate, rituximab, rolapitant, romidepsin, romiplostim, romurtide, rucaparib, samarium (153Sm) lexidronam, sargramostim, sarilumab, satumomab, secretin, siltuximab, sipuleucel-T, sizofiran, sobuzoxane, sodium glycididazole, sonidegib, sorafenib, stanozolol, streptozocin, sunitinib, talaporfin, talimogene laherparepvec, tamibarotene, tamoxifen, tapentadol, tasonermin, teceleukin, technetium (99mTc) nofetumomab merpentan, 99mTc-HYNIC-[Tyr3]-octreotide, tegafur, tegafur + gimeracil + oteracil, temoporfin, temozolomide, temsirolimus, teniposide, testosterone, tetrofosmin, thalidomide, thiotepa, thymalfasin, thyrotropin alfa, tioguanine, tisagenlecleucel, tocilizumab, topotecan, toremifene, tositumomab, trabectedin, trametinib, tramadol, trastuzumab, trastuzumab emtansine, treosulfan, tretinoin, trifluridine + tipiracil, trilostane, triptorelin, trametinib, trofosfamide, thrombopoietin, tryptophan, ubenimex, valatinib, valrubicin, vandetanib, vapreotide, vemurafenib, vinblastine, vincristine, vindesine, vinflunine, vinorelbine, vismodegib, vorinostat, vorozole, yttrium-90 glass microspheres, zinostatin, zinostatin stimalamer, zoledronic acid, zorubicin.

The term "combination" in the present invention is used as known to persons skilled in the art, it being possible for said combination to be a fixed combination, a non-fixed combination or a kit-of-parts.

A "fixed combination" in the present invention is used as known to persons skilled in the art and is defined as a combination wherein, for example, a first active ingredient, such as one or more compounds of general formula (I) of the present invention, and a further active ingredient are present together in one unit dosage or in one single entity. One example of a "fixed combination" is a pharmaceutical composition wherein a first active ingredient and a further active ingredient are present in admixture for simultaneous administration, such as e.g. in a formulation. Another example of a "fixed combination" is a pharmaceutical combination wherein a first active ingredient and a further active ingredient are present in one unit without being in admixture.

A non-fixed combination or "kit-of-parts" in the present invention is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.

In another embodiment the invention relates to a pharmaceutical composition comprising modification C of the compound of formula (I) and optionally an amount of other forms of the compound of formula (I) in combination with another anti-cancer agent as listed above.

## Pharmaceutical compositions

It is possible for the crystalline form C of the compound of formula (I) according to the present invention to have systemic and/or local activity. For this purpose, it can be administered in a suitable manner, such as, for example, via the oral, parenteral, pulmonary, nasal, sublingual, lingual, buccal, rectal, vaginal, dermal, transdermal, conjunctival, otic route or as an implant or stent.

For these administration routes, it is possible for the crystalline form C of the compound of formula (I) according to the present invention to be administered in suitable administration forms.

For oral administration, it is possible to formulate the crystalline form C of the compound of formula (I) according

to the present invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compound according to the invention in crystalline and/or amorphised and/or dissolved form into said dosage forms.

Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.

Examples which are suitable for other administration routes are pharmaceutical forms for inhalation [inter alia powder inhalers, nebulizers], nasal drops, nasal solutions, nasal sprays;
tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.

The crystalline form of the compound of formula (I) can be incorporated into the stated administration forms. This can be effected in a manner known per se by mixing with pharmaceutically suitable excipients. Pharmaceutically suitable excipients include, inter alia,

- fillers and carriers (for example cellulose, microcrystalline cellulose (such as, for example, Avicel®), lactose, mannitol, starch, calcium phosphate (such as, for example, Di-Cafos®)),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette®), sorbitan fatty acid esters (such as, for example, Span®), polyoxyethylene sorbitan fatty acid esters (such as, for example, Tween®), polyoxyethylene fatty acid glycerides (such as, for example, Cremophor®), polyoxethylene fatty acid esters, polyoxyethylene fatty alcohol ethers, glycerol fatty acid esters, poloxamers (such as, for example, Pluronic®),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol®); alginates, gelatine),
- disintegrants (for example modified starch, carboxymethylcellulose-sodium, sodium starch glycolate (such as, for example, Explotab®), cross- linked polyvinylpyrrolidone, croscarmellose-sodium (such as, for example, AcDiSol®)),
- flow regulators, lubricants, glidants and mould release agents (for example magnesium stearate, stearic acid, talc, highly-disperse silicas (such as, for example, Aerosil®)),
- coating materials (for example sugar, shellac) and film formers for films or diffusion membranes which dissolve rapidly or in a modified manner (for example polyvinylpyrrolidones (such as, for example, Kollidon®), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit®)),

- capsule materials (for example gelatine, hydroxypropylmethylcellulose),
- synthetic polymers (for example polylactides, polyglycolides, polyacrylates, polymethacrylates (such as, for example, Eudragit®), polyvinylpyrrolidones (such as, for example, Kollidon®), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

The present invention furthermore relates to a pharmaceutical composition comprising at least the crystalline form C of the compound of formula (I) according to the present invention, conventionally together with one or more pharmaceutically suitable excipient(s), and their use according to the present invention.

Furthermore in some embodiments the invention relates to a pharmaceutical composition comprising modification C of the compound of formula (I) and optionally further pharmaceutically acceptable excipients.

In another embodiment the invention relates to the pharmaceutical composition comprising modification C of the compound of the formula (I) mainly and no significant fractions of another form of the compound of formula (I) and optionally further pharmaceutically acceptable excipients

In yet other embodiments the invention relates to the pharmaceutical composition comprising modification C of the compound of formula (I) mainly and less than 15 % of another form or other forms of the compound of formula (I) and optionally further pharmaceutically acceptable excipients.

In further embodiments the invention relates to the pharmaceutical composition comprising modification C of the compound of the formula (I) wherein the amount of another form or other forms of the compound of formula (I) is less than 10 %.

In further embodiments the invention relates to the pharmaceutical composition comprising modification C of the compound of the formula (I), wherein the amount of another form or other forms of the compound of formula (I) is less than 5 %.

## Dosage of the pharmaceutical compositions of the present invention:

Based upon laboratory techniques known to evaluate compounds useful for the treatment of disorders, by pharmacological assays for the determination of treatment of the conditions identified above in mammals, and by comparison of these results with the results of known medicaments that are used to treat these conditions, the effective dosage of the compound of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered by injection, including intravenous, intramuscular, subcutaneous and parenteral injections, and use of infusion techniques, rectal dosage, vaginal dosage regimen, topical dosage regimen, transdermal dosage, inhalation dosage or oral dosage will generally range from about 0.01 to 200mg/kg body weight per day. A unit dosage may contain from about 0.001g to about 1.500g of active ingredient, and can be administered one or more times per day or less than once a day. In one aspect the unit dosage contains 10mg of active ingredient.

Of course the specific initial and continuing dosage regimen for each patient will vary according to the nature

and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

The weight data in the tests and examples which follow are, unless stated otherwise, percentages by weight; parts are parts by weight. Solvent ratios, dilution ratios and concentration data of liquid/liquid solutions are based on each case on the volume.

## Working examples

### General Methods:

#### Differential Scanning Calorimetry (DSC) /Thermogravimetry (TG) or Thermogravimetry Analysis (TGA)

a) Preparation of the sample
There was no sample preparation.
b) Measurement details

1) Differential scanning calorimetry (DSC) was performed with a Perkin Elmer thermal analysis system Diamond DSC and DSC 8000. The calorimeter was purged with nitrogen gas. 2 - 5 mg of each sample was placed into a perforated aluminum crucible and heated at a rate of 2° or 20 °K/min from -0 °C to 160°C. There was no sample preparation.
2) Thermogravimetric analysis (TGA) was performed with a Perkin Elmer thermal analysis system TGA7 .The instrument was purged with nitrogen gas. Approximately 2 - 5 mg of each sample was placed into an open platinum crucible and heated at a heating rate of 10 K/min from 35 °C to 160°C.

#### XRPD

Method A:

a) Preparation of the sample
There was no sample preparation.
b) Measurement details
X-Ray diffraction patterns were recorded at room temperature using XRD-Transmissions-Diffractometer X'Pert PRO (PANalytical) (radiation Cu K alpha 1, wavelength 1.5406 Å, 40mA, 40kV). There was no sample preparation. All X-Ray reflections are quoted as °2Θ (theta) values (peak maxima) with a resolution of ±0.2°.

Method B

X-ray powder diffraction (XRPD) data were recorded on a STOE STADI P diffractometer using monochromatized CuKa1-radiation, a position sensitive detector, at generator settings of 40 kV and 40 mA. The samples were collected in transition mode, being prepared as a thin layer between two foils. The scanning range was between 2 ° and 40 ° 2 theta with a 0.5° step at 15 sec/step.

#### IR

a) Preparation of the sample
There is no sample preparation
b) Measurement details
IR spectra were acquired on a IR-Spectrometer Bruker Tensor 37 with ATR-unit. Each spectrum represents 64 coadded scans with a resolution of 2 cm-1 in the following measuring range of 550-4000 cm-1.

**Raman**

a) Preparation of the sample
There was no sample preparation
b) Measurement details
Raman spectra were recorded at room temperature using FT-Raman-spectrometers (model RFS 100 and MultiRam) from Bruker at 25°C using a 1064 nm NdYAG Laser at. Resolution was 2 cm-1. Measurements were performed in glass vials or aluminium discs. Each spectrum represents 64 coadded scans with a resolution of 2 cm-1 in the following measuring range of 100 - 3500.

**LCMS (method 1): HSST3**

Instrument: Waters ACQUITY SQD UPLC system; column: Waters Acquity UPLC HSS T3 1.8 μm 50 × 1 mm; eluent A: 1 l water + 0.25 ml 99% formic acid, eluent B: 1 l acetonitrile + 0.25 ml 99% formic acid; gradient: 0.0 min 90 % A → 1.2 min 5% A → 2.0 min 5% A; flow rate: 0.40 ml/min; UV detection: 208 - 400 nm.

**GCMS (method 2): DSQ-II**

Instrument: Thermo Scientific DSQII, Thermo Scientific Trace GC Ultra system; column: Restek RTX-35MS, 15 m × 200 μm × 0.33 μm; constant helium flow: 1.20 ml/min; oven: 60°C; inlet: 220°C; gradient: 60°C, 30°C/min → 300°C (hold time 3.33 min).

**Chiral HPLC (method 3):**

System: High performance liquid chromatograph equipped with gradient pumps, UV detector & attached with data recorder and integrator software; column: Chiralpak IA (250*4.6 mm, 5μm); flow: 1 mL/min; column temperature: 40°C; injection volume 10μL, detection 292 nm, run time: 15 min; mobile phase: n-heptane / 2-propanole 90/10 (v/v).

**Example 1: Manufacture of modifications of (6S)-5-[4'-Fluoro-2-(trifluoromethyl)biphenyl-4-yl]-6-methyl-3,6-dihydro-2H-1,3,4-oxadiazin-2-one - the compound of formula (I) -** in large scale

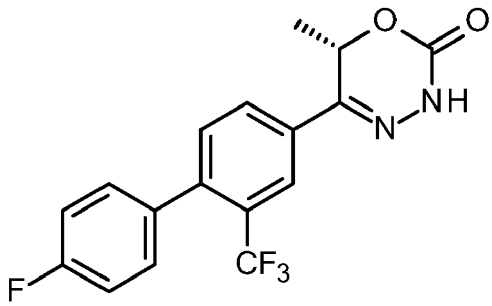

(I)

The compound of formula (I) can be prepared according to the procedures disclosed in WO2019/025562.
An alternative process can be performed according to the following examples 1.1 - 1.5:

**Example 1.1** - 4-Bromo-4'-fluoro-2-(trifluoromethyl)[biphenyl]

To 166,3 kg toluene and 96,0 kg water were added 24,0 kg (68,4 mol) commercially available 4-bromo-1-iodo-2-(trifluoromethyl)benzene, 12,5 kg (89,3 mol) (4-fluorophenyl)boronic acid and 21,7 kg (204,7 mol) sodium carbonate. To the mixture was added 250 g (0,34 mol) Pd(dppf)Cl2 and the resulting biphasic solution was heated to 50-60°C for

6 h. The mixture was cooled down to 20°C and the phases were separated. The aqueous phase was extracted with 41,6 kg toluene and the toluene phases were combined. The organic phase was filtered through silica gel (12 Kg) to remove Pd-residues. Subsequently, the organic solvent was removed in vacuo to give crude product, which was distilled (1 mbar, 120°C) to give 16,6 kg 4-bromo-4'-fluoro-2-(trifluoromethyl)[biphenyl] as a colorless liquid in 76 % yield.

1H NMR (600 MHz, DMSO-d6) δ ppm: 7,29 (m, 2 H), 7,36 (t, J=7,63 Hz, 3 H), 7,94 (dd, J1=8,22 Hz, J2=1,96 Hz, 1 H), 8,00 (d, J=1,96 Hz, 1 H).

GCMS (method 2): Rt = 4,69 min; MS m/z = 320, 318 (M-H)+, 219, 170, 110.

**Example 1.2** - (2S)-1-[4'-Fluoro-2-(trifluoromethyl)[biphenyl]-4-yl]-2-(tetrahydro-2H-pyran-2-yloxy)-propan 1-one

To 1,80 kg (5,64 mol) 4-bromo-4'-fluoro-2-(trifluoromethyl)[biphenyl] were added 1,60 kg THF. At 20 °C, 3,30 kg (6,77 mol) Isopropylmagnesium chloride 2M in THF were added to the above prepared solution over 45 min. During preparation of the Grignard solution, the reaction mixture gets heated to 28°C and the mixture is stirred at 28°C for 5 h. The mixture is cooled to 0 - 4°C.

A solution of 1,53 kg (6,28 mol) (2S)-1-(morpholin-4-yl)-2-(tetrahydro-2H-pyran-2-yloxy)propan-1-one prepared according to J. Pesti et al., Org. Process Res. Dev. 2009, 13, 716-728; (using the alternative enantiomer as starting material) in 3,20 kg THF is added to the above prepared Grignard reagent at 0 - 4°C within 30 min. The mixture is heated to 20 °C and is stirred for 30 min at 20°C.

A citric acid solution in water (prepared from 2,70 kg citric acid and 10,10 kg water) is added to the mixture at 20°C. The addition takes about 60 min.

11,34 kg Ethyl acetate are added to the quenched mixture and the two-phasic mixture is stirred for 10 min at 20 °C. The phases are separated and the lower aqueous phase is discarded.

To the upper organic phase (approx. 24 L) is added 6,50 kg saturated NaCl solution in water and the mixture is stirred for 10 min at 20°C. The phases are separated and the lower aqueous phase is discarded.

The organic solvent of the upper organic phase (approx. 22,5 L) is distilled off (approx. 17,5 L solvent is distilled off) at 50°C and 80 mbar to give an oil. 8,10 kg Ethyl acetate are added to the oil and the solvent is removed at 50°C and 80 mbar (approx. 9 L solvent is distilled off). To the residue is added 9,10 kg Ethyl acetate.

The prepared solution of (2R)-1-[4'-fluoro-2-(trifluoromethyl)[biphenyl]-4-yl]-2-(tetrahydro-2H-pyran-2-yloxy)propan-1-one in Ethyl acetate (approx. 12 kg solution) is used in the next step (synthesis of (2R)-1-[4'-fluoro-2-(trifluoromethyl)[biphenyl]-4-yl]-2-hydroxypropan-1-one).

1H NMR (600 MHz, DMSO-d6) δ ppm: 0,99 (m, 1 H), 1,25 (m, 1 H), 1,38 (d, J=6,65 Hz, 3 H), 1,44 (d, J=6,85 Hz, 3 H), 1,48 (m, 5 H), 1,69 (m, 5 H), 3,47 (m, 1 H), 3,55 (d, J=4,89 Hz, 1 H), 3,63 (td, J1=7,43 Hz, J2=7,43 Hz, J3=3,52 Hz, 1 H), 3,81 (m, 1 H), 4,67 (t, J=3,33 Hz, 1 H), 4,82 (dd, J1=5,09 Hz, J2=2,15 Hz, 1 H), 5,09 (q, J=6,65 Hz, 1 H), 5,24 (q, J=7,04 Hz, 1 H), 7,32 (td, J1=8,90 Hz, J2=0,98 Hz, 4 H), 7,42 (m, 4 H), 7,60 (dd, J1=7,92 Hz, J2=4,79 Hz, 2 H), 8,34 (m, 3 H), 8,49 (s, 1 H). GCMS (method 2): Rt = 3,67 min; MS m/z = 240, 219, 201, 170.

**Example 1.3** - (2S)-1-[4'-Fluoro-2-(trifluoromethyl)[biphenyl]-4-yl]-2-hydroxypropan-1-one

To 12 kg solution of (2S)-1-[4'-fluoro-2-(trifluoromethyl)[biphenyl]-4-yl]-2-(tetrahydro-2H-pyran-2-yloxy)propan-1-one in Ethyl acetate (see example 2) is added 1,09 kg (11,3 mol) of methanesulfonic acid at 20°C. The mixture is stirred for 2 h at 20°C. 8 kg water are added to the dark mixture at 20°C and the mixture is stirred for 10 min.

The phases are separated and the aqueous phase (approx. 9 kg) is extracted with 3 kg Ethyl acetate. The Ethyl acetate phases from both extractions are combined and 6 kg saturated NaCl solution in water is added. The mixture is stirred for 10 min. The phases are separated and the lower aqueous phase is discarded.

The upper organic phase (approx. 16 L) is distilled off at 50 °C and 80 mbar to give an oil. 5 kg Ethyl acetate is added to the oil and the solvent is distilled off at 50 °C and 80 mbar to give an oil.

The resulting oil is purified by flash chromatography (SiO2) with Heptane/Ethyl acetate (2:1) or can be alternatively distilled (< 0,1 mbar, 100 °C) to give the purified (2S)-1-[4'-fluoro-2-(trifluoromethyl)- [biphenyl]-4-yl]-2-hydroxypropan-1-one (approx. 1,2 kg) as a slightly yellow oil in 68% yield over 2 steps.

1H NMR (600 MHz, DMSO-d6) δ ppm: 1,34 (d, J=6,85 Hz, 3 H), 5,07 (quin, J=6,65 Hz, 1 H), 5,61 (d, J=6,46 Hz, 1 H), 7,32 (t, J=8,80 Hz, 2 H), 7,41 (dd, J1=8,51 Hz, J2=5,58 Hz, 2 H), 7,58 (d, J=8,02 Hz, 1 H), 8,32 (d, J=8,02 Hz, 1 H), 8,37 (s, 1 H).

The two steps described here (synthesis of (2S)-1-[4'-fluoro-2-(trifluoromethyl)[biphenyl]-4-yl]-2-(tetrahydro-2H-pyran-2-yloxy)propan-1-one and synthesis of (2S)-1-[4'-fluoro-2-(trifluoromethyl)[biphenyl]-4-yl]-2-hydroxypropan-1-one) were performed several times and batches were combined to get 3,05 kg of (2S)-1-[4'-fluoro-2-(trifluoromethyl)[biphenyl]-4-yl]-2-hydroxypropan-1-one to perform the next step (Example 4) in reasonable batch size.

**Example 1.4 - Methyl (2Z)-2-{(2S)-1-[4'-fluoro-2-(trifluoromethyl)[biphenyl]-4-yl]-2-hydroxy-propylidene}hydrazinecarboxylate**

To 3,05 kg (9,76 mol) (2S)-1-[4'-fluoro-2-(trifluoromethyl)[biphenyl]-4-yl]-2-hydroxypropan-1-one is added 21,2 kg ethanol. 0,88 kg (9,76 mol) methyl hydrazine carboxylate as well as 0,1 L of HCl 0,1M in water (0,01 mol) are added at 20 °C. The mixture is heated to reflux for 18 h.

Approx. 20 L of solvent are distilled off at 90 °C and 7,4 kg Diisopropyl ether is added.

Approx. 9 L of solvent are distilled off at 80-90 °C and 7,4 kg Diisopropyl ether is added.

Approx. 12 L of solvent are distilled off at 80-90 °C and 7,4 kg Diisopropyl ether is added.

The mixture is cooled to 2 °C over 60 min and is stirred for approx. 8 h at 2 °C.

The precipitated product is filtered and washed twice with each time 2 kg cold Diisopropyl ether.

The wet product is dried at 50 °C in-vacuo to give 2,01 kg of methyl (2Z)-2-{(2S)-1-[4'-fluoro-2-(trifluoromethyl)[biphenyl]-4-yl]-2-hydroxypropylidene}hydrazinecarboxylate as a white solid in 54% yield.

1H NMR (400 MHz, DMSO-d6) $\delta$ ppm: 1,38 (d, J=6,72 Hz, 3 H), 3,72 (s, 3 H), 5,27 (dd, J1=6,79 Hz, J2=3,36 Hz, 1 H), 6,38 (d, J=3,06 Hz, 1 H), 7,36 (m, 5 H), 8,00 (d, J=8,34 Hz, 1 H), 8,12 (s, 1 H), 11,06 (s, 1 H).

LCMS (method 1): Rt = 1,04 min; MS (ESIpos): m/z = 385 (M+H)+

**Example 1.5** - (6S)-5-[4'-Fluoro-2-(trifluoromethyl)[biphenyl]-4-yl]-6-methyl-3,6-dihydro-2H-1,3,4-oxadiazin-2-one (compound of formula (I))

To 15,8 kg ethanol is added 2,00 kg (5,20 mol) methyl (2Z)-2-{(2S)-1-[4'-fluoro-2-(trifluoromethyl) [biphenyl]-4-yl]-2-hydroxypropylidene}hydrazinecarboxylate at 20 °C.

464 g sodium hydroxide solution (45% in water) is added and the mixture is stirred at 20 °C for 1 h. Subsequently, 312 g acetic acid is added and the mixture is stirred at 20 °C for 30 min.

After filtration (final processing), 21,0 kg purified water is added to the mixture at 20°C. A suspension of seed crystals*) (modification C) in ethanol/water (1:1) was added. The mixture is stirred at 20 °C for 8 h. The crystallized product is filtered, washed twice each time with 2 L water/ethanol (1:1) and dried at 50°C in-vacuo to give 1,66 kg (6S)-5-[4'-fluoro-2-(trifluoromethyl)[biphenyl]-4-yl]-6-methyl-3,6-dihydro-2H-1,3,4-oxadiazin-2-one as a white solid in 91% yield.

*) seed crystals could be produced by the following method:
0,5 g of (6S)-5-[4'-fluoro-2-(trifluoromethyl)[biphenyl]-4-yl]-6-methyl-3,6-dihydro-2H-1,3,4-oxadiazin-2-one (obtained as modification A and B in analogy to e.g. published methods) is added to 10 mL of a 1:1 mixture of water/ethanol (approx.. 5 w.%). A suspension of (6S)-5-[4'-fluoro-2-(trifluoromethyl) [biphenyl] -4-yl] -6-methyl-3,6-dihydro-2H-1,3,4-oxadiazin-2-one in ethanol/water (1:1) is formed. After stirring the suspension for 16 h at 30 °C, the suspension is filtered. The solid is dried to obtain (6S)-5-[4'-fluoro-2-(trifluoromethyl)[biphenyl]-4-yl]-6-methyl-3,6-dihydro-2H-1,3,4-oxadiazin-2-one in modification C. The first seed crystals were obtained following the route described in example 1.8

1H NMR (500 MHz, DMSO-d6) $\delta$ ppm: 1,49 (d, J=6,94 Hz, 3 H), 5,96 (q, J=6,91 Hz,1 H), 7,31 (t, J=7,96 Hz, 2 H), 7,40 (t, J=6,48 Hz, 2 H), 7,53 (d, J=8,04 Hz, 1 H), 8,06 (dd, J1=8,12 Hz, J2=1,50 Hz, 1 H), 8,17 (s, 1 H), 11,32 (s, 1 H).

LCMS (method 1): Rt = 1,02 min; MS (ESIneg): m/z = 351 (M-H)-
Chiral HPLC (method 3): Rt (R-enantiomer) = 10,5 min; Rt (S-enantiomer) = 11,5 min.
ee (S-enantiomer) > 99,5%.

Modification C was obtained in example 1.5 as shown in the XRPD diagram of FIG. 1 using Method B.

Optionally a micronization step can be performed.

In analogy to Example 1.5, five batches**) were prepared and subsequently all batches were combined and micronized twice in a row to give 5,60 kg of micronized material. Here, details for micronization are given:
Five batches of (6S)-5-[4'-fluoro-2-(trifluoromethyl)[biphenyl]-4-yl]-6-methyl-3,6-dihydro-2H-1,3,4-oxadiazin-2-one were prepared according to Example 1.5 and were combined. The combined batches were micronized twice in a row using an air jet mill.
a) Used Device:

| | |
|---|---|
| Used Device: Mill | 200 mm spiral jet mill (Bayer design) |
| Dosing device (Type) | vibrating chute |
| Grinding chamber lining (material) | PTFE |
| Injector motive nozzle (diameter) | 2,9 mm |

(continued)

| | |
|---|---|
| Used Device: Mill | 200 mm spiral jet mill (Bayer design) |
| Grinding nozzle (diameter) | 2,2 mm 32° |
| Collecting nozzle (diameter) | 8 mm PTFE |
| Outlet opening (diameter) | 28,3 mm PTFE |

**b) Grinding Parameters**

| Grinding Parameter | | Actual value |
|---|---|---|
| Injector air pressure | [bar] | 4,3 |
| Grinding air pressure | [bar] | 6 |
| Product throughput | [g/min] | 100 |
| Total grinding time | [min] | 68 |

**c) The micronized product obtained has the following properties:**

Particle Size distribution (X10/X50/X90): 1,2/4/10 μm.
The instrumental set-up for particle size analysis:

Laser diffraction pattern analyzer (Sympatec HELOS, H1970 & RODOS, R3:0.5/0.9....175μm),
100 mm focal length,
Dry dispersion (RODOS) at 4 bar,
Mathematical model (LD 5.9.0.0) - Sympatec Code, basis Fraunhofer Diffraction

**) A probe of the product of one of these five batches was used in example 4 and 5 as probe 2. The XRPD spectrum is shown in FIG. 2 which is obtained using Method B.

Example 1.6 - Preparation of modification A

If the seed crystals mentioned in example 1.5 were dissolved completely or not added at all, modification A was spontaneously obtained by following the same procedure as described above under 1.5.

To 15,8 kg ethanol is added 2,00 kg (5,20 mol) methyl (2Z)-2-{(2S)-1-[4'-fluoro-2-(trifluoromethyl) [biphenyl]-4-yl]-2-hydroxypropylidene}hydrazinecarboxylate at 20 °C.

464 g sodium hydroxide solution (45% in water) is added and the mixture is stirred at 20 °C for minimum 4 h. Subsequently, 312 g acetic acid is added and the mixture is stirred at 20 °C for 30 min. After filtration (final processing), 21,0 kg purified water is added over 3 h to the mixture at 20°C. A suspension of seed crystals*) (modification C) in ethanol/water (1:1) was added during addition of the water. The mixture is stirred at 20 °C for 8 h. A further suspension of seed crystals*) (modification C) in ethanol/water (1:1) was added. The mixture is stirred at 20 °C for 8 h. The mixture was heated to 60 °C and then cooled to 50 °C. A further suspension of seed crystals*) (modification C) in ethanol/water (1:1) was added. The mixture was cooled to 20 °C using a linear 2 h ramp. The mixture was stirred at 20 °C for 8 h. The crystallized product is filtered, washed twice each time with 2 L water/ethanol (1:1) and dried at 50 °C in-vacuo to give 1,24 kg (6S)-5-[4'-fluoro-2-(trifluoromethyl)[biphenyl]-4-yl]-6-methyl-3,6-dihydro-2H-1,3,4-oxadiazin-2-one, modification A, as a white solid in 67% yield.

The respective XRPD diagram is illustrated by FIG. 3 which was obtained using Method B.

The product as obtained was used in examples 4 and 5 as probe 1.

**Example 1.7** - Alternatively to example 1.5 the following procedure could also be followed, surprisingly directly leading to modification B:

Crude methyl (2Z)-2-{(2S)-1-[4'-fluoro-2-(trifluoromethyl) [biphenyl]-4-yl]-2-hydroxypropylidene}hydrazinecarboxylate (approx. 13,54 g in 100 mL methanol) was stirred at room temperature and 26,1 mL of sodium methylate (5,4 M in methanol) were added at room temperature. The mixture was stirred at room temperature overnight. 8,46 g of acetic

acid were added and the mixture was stirred for 10 min at room temperature. The mixture was concentrated in vacuo. Saturated aqueous sodium chloride solution was added and the mixture was extracted with ethyl acetate. The organic phase was dried and concentrated in vacuo. 13,00 g of a crude oil were obtained. The crude oil was purified via column chromatography twice ($SiO_2$, hexane/ethyl acetate 100:0 to 70:30). The fractions containing the product were concentrated in vacuo giving 7,8 g of product, which were suspended in 140 mL of ethanol/water 1:1 and stirred for 30 min at room temperature. The obtained solid was filtered off and suspended again in 140 mL of ethanol/water 1:1 and stirred for 30 min at room temperature. The obtained solid was filtered off and dried overnight at 50 °C. 4,20 g of (6S)-5-[4'-fluoro-2-(trifluoromethyl)[biphenyl]-4-yl]-6-methyl-3,6-dihydro-2H-1,3,4-oxadiazin-2-one were obtained.

As a result modification B was obtained which is illustrated by the XRPD of FIG. 4 (obtained by using Method A), the IR-Spectrum of FIG. 11 and the Raman spectrum of FIG. 14

**Example 1.8** First preparation of modification C

1) In a vessel was charged (6S)-5-[4-chloro-3-(trifluoromethyl)phenyl]-6-methyl-3,6-dihydro-2H-1,3,4-oxadiazin-2-one (7.65 g, 25.10 mmol) obtained following the route described under "Intermediate 74" as starting material for example 135 of WO2019/025562, 4-fluorophenylboronic acid (5.27 mg, 37.64 mmol), 2-(Dicyclohexylphosphino)-2',4',6'-triisopropylbiphenyl (XPhos, 718 mg, 1.51 mmol) and $K_2CO_3$ (7.18 g, 50.19 mmol) and Chloro(2-dicyclohexylphosphino-2',4',6'-triisopropyl-1,1'-biphenyl) [2-(2'-amino-1,1'-biphenyl)]palladium(II), X-Phos aminobiphenyl palladium chloride precatalyst (XPhos-Pd-G2, 1.18 g, 0.75 mmol) in 1,4-dioxane (115 mL) and water (38.4 mL). The resultant mixture was degassed via nitrogen-filled balloon for 5 mins, with subsequent heating at 80 °C for 2 hours. After this time, the mixture was diluted with Ethyl acetate (50 mL) and washed with saturated aqueous NaCl solution (3 $\times$ 20 mL), dried ($Na_2SO_4$), filtered and concentrated at reduced pressure. The residue was purified via Biotage Isolera chromatography (using a gradient of eluents; 1:0 to 1:1 Heptane:Ethyl acetate). The desired fractions were combined, concentrated in vacuo and triturated with tert-butyl methyl ether/Heptane (1:1) with the insoluble material isolated by suction filtration and dried in vacuo to give a grey powder (6.45 g, 95.88 %ee). This material was suspended in 40%Ethanol/Water and warmed to facilitate dissolution. Once the majority of the material was dissolved, the solution was filtered through a cotton plug and allowed to cool to room temperature and stand at room temperature for 60 h. After this time, the crystallized material was isolated by suction filtration and dried in vacuo to afford the product as a colourless crystalline solid (4.48 g, 89.1 %ee). This material was suspended in 40%Ethanol/Water and warmed to facilitate dissolution, with the solution was filtered through a cotton plug and allowed to cool to room temperature and stand at room temperature for 16 h, with the crop of crystalline material isolated to afford (6S)-5-[4'-fluoro-2-(trifluoromethyl)[biphenyl]-4-yl]-6-methyl-3,6-dihydro-2H-1,3,4-oxadiazin-2-one in 39% yield (3.46 g, 93.78 %ee). This process lead to modification B.

The XRPD of Modification B as obtained is illustrated in FIG. 5 (obtained by using Method A).

2) In a flask (6S)-5-[4'-fluoro-2-(trifluoromethyl)[biphenyl]-4-yl]-6-methyl-3,6-dihydro-2H-1,3,4-oxadiazin-2-one (2.00 g, (93.78 %ee) of the product as obtained under 1) of example 1.8, which was stored at room temperature for about 7 month, was suspended in 20 mL of water and 20 mL of ethanol and stirred at room temperature for 30 min. The solid was collected by filtration and the collected solids were again suspended in 20 mL of water and 20 mL of ethanol and stirred at room temperature for 30 min. The remaining crystalline solid was collected by filtration again, dried in a vacuum oven at 50°C overnight to yield 1.62g of the title compound (6S)-5-[4'-fluoro-2-(trifluoromethyl)[biphenyl]-4-yl]-6-methyl-3,6-dihydro-2H-1,3,4-oxadiazin-2-one with 99.7% ee (99% purity by LCMS and H-NMR). Surprisingly the obtained product had another modification, which was modification C. The XRPD is shown in FIG. 6 (obtained by using Method A). Its reflections are shown below:

| Reflections [°2Theta] |
|---|
| 8.3 |
| 11.8 |
| 11.9 |
| 15.1 |
| 15.1 |
| 17.3 |
| 18.8 |
| 18.9 |
| 20.9 |

(continued)

| Reflections [°2Theta] |
|---|
| 21.1 |
| 22.5 |
| 22.6 |
| 23.6 |
| 26.8 |
| 28.0 |
| 28.5 |
| 29.6 |
| 29.8 |
| 30.4 |
| 30.5 |
| 31.5 |
| 31.6 |
| 33.0 |
| 34.9 |
| 35.1 |
| 35.8 |
| 36.2 |
| 36.6 |
| 36.9 |
| 37.3 |
| 37.6 |

## Example 2 Crystallization experiments

2.1 Preparation of Modification A:

~ 400 mg of the compound of formula (I) as obtained in example 1.7 was dissolved in 40 mL of the below mentioned solvents at room temperature followed by heating under reflux. The resulting solutions were quartered and stored

- at ambient conditions
- in a refrigerator
- in a freezer

until evaporation was completed whereas the last quarter was

- precipitated with water and evaporated under ambient conditions.

For acetone and acetonitrile after one day the precipitate was filtered and dried at room temperature whereas the precipitate from ethanol could not be filtered and hence the suspension was allowed to dry at room temperature.

The respective XRPD diagram is shown in FIG. 7 (obtained by using Method A), the IR- und Raman-spectrum is shown in FIG. 10 and FIG. 13 respectively.

| Solvent | Conditions | Resulting Polymorph |
|---|---|---|
| Acetone | Evaporation at ambient conditions | Mod. A |
| Acetone | Evaporation in a refrigerator | Mod. A *) |
| Acetone + Water | Evaporation at ambient conditions | Mod. A |
| Acetonitrile | Evaporation at ambient conditions | Mod. A |
| Acetonitrile | Evaporation in a refrigerator | Mod. A |
| Acetonitrile + Water | Evaporation at ambient conditions | Mod. A |
| Ethanol | Evaporation at ambient conditions | Mod. A |
| Ethanol | Evaporation in a refrigerator | Mod. A |
| Ethanol | Evaporation in a freezer | Mod. A |
| *) probe used for obtaining the XRPD diagram of FIG. 7 and the IR- and Raman spectra of FIG.s 10 and 13. | | |

2.2 Preparation of Modification B:

400 mg of the compound of formula (I) as obtained in example 1.7 was dissolved in 40 mL of ethanol/water at room temperature followed by heating under reflux. The resulting solution was quartered and one quarter was stored at ambient conditions until evaporation was completed For the condition mentioned below Modification B was obtained.

The respective XRPD diagram is shown in FIG. 8 (obtained by using Method A).

| Solvent | Conditions | Resulting Polymorph |
|---|---|---|
| Ethanol + Water | Evaporation at ambient conditions | Mod. B *) |
| *) probe used for obtaining the XRPD diagram of FIG 8. | | |

The IR- and Raman spectra of Modification B shown in FIG. 11 and FIG. 14 were obtained from a sample of example 1.7

2.3 Preparation of Modification C

In a slurry experiment 107mg of the compound of formula (I) as obtained in example 1.7 was suspended in 0,5mL of solvent, seed crystals of example 1.8 2) were added to the resulting suspension and it was shaken for a week at 25°C in a Themomix. After half of the time again 0.5mL of solvent were added and shaked for a week at 25°C in a Themomix. The mixture was filtered and dried under room conditions.

| Solvent | Conditions | Resulting Polymorph |
|---|---|---|
| n-Heptan | Slurry experiment | Mod. C |
| Ethanol/water (1:1) | Slurry experiment | Mod. C *) |
| *) probe used for obtaining the XRPD diagram of FIG. 9 (obtained by using Method A), the IR- und Raman-spectra as shown in FIG. 12 and FIG. 15 respectively. | | |

**Example 3: Physical characterization of modifications of (6S)-5-[4'-Fluoro-2-(trifluoromethyl)biphenyl-4-yl]-6-methyl-3,6-dihydro-2H-1,3,4-oxadiazin-2-one, compound of formula (I) by different methods**

3.1 XRPD - Reflections and related figures

They are obtained following the method as described under General methods above as Method A:

| Reflections (Peak maxima) [2 Theta] | | |
|---|---|---|
| **Modification A FIG. 7** | **Modification B FIG. 8** | **Modification C FIG. 9** |
| 10.2 | 9.1 | 11.8 |
| 11.8 | 10.9 | 11.9 |
| 12.3 | 11.8 | 15.1 |
| 12.8 | 12.4 | 17.2 |
| 13.2 | 12.7 | 18.8 |
| 13.5 | 12.8 | 18.9 |
| 13.9 | 14.4 | 21.1 |
| 14.3 | 15.1 | 22.6 |
| 14.4 | 15.6 | 22.6 |
| 15.2 | 15.8 | 26.8 |
| 15.4 | 16.0 | 27.9 |
| 15.9 | 16.7 | 28.5 |
| 16.3 | 16.9 | 29.6 |
| 16.7 | 18.3 | 29.8 |
| 17.0 | 18.9 | 30.4 |
| 17.4 | 19.4 | 31.6 |
| 17.8 | 20.5 | 33.0 |
| 18.2 | 20.8 | |
| 19.0 | 21.5 | |
| 19.3 | 21.9 | |
| 20.5 | 22.2 | |
| 20.7 | 23.3 | |
| 21.0 | 23.4 | |
| 21.5 | 23.7 | |
| 22.0 | 24.2 | |
| 22.2 | 24.6 | |
| 23.2 | 25.8 | |
| 23.9 | 26.1 | |
| 25.6 | 26.8 | |
| 26.0 | 27.2 | |
| 26.2 | 27.5 | |
| 26.8 | 28.3 | |
| 27.0 | 29.1 | |
| 27.4 | 30.4 | |
| 28.6 | 33.2 | |
| 29.1 | | |
| 30.3 | | |
| 35.1 | | |

(continued)

| Reflections (Peak maxima) [2 Theta] | | |
|---|---|---|
| **Modification A FIG. 7** | **Modification B FIG. 8** | **Modification C FIG. 9** |
| 35.7 | | |
| 36.7 | | |

3.2 IR bands obtained following the method as described under General Methods above

| IR Bands [Peak maxima in cm-1] | | |
|---|---|---|
| **Modification A FIG. 10** | **Modification B FIG. 11** | **Modification C FIG. 12** |
| 3261 | 3260 | 3218 |
| 3170 | 3164 | 3148 |
| 3077 | 3078 | 3070 |
| 2985 | 2984 | 3009 |
| 2962 | 2963 | 2913 |
| 2908 | 2894 | 1730 |
| 1732 | 1732 | 1617 |
| 1698 | 1697 | 1607 |
| 1628 | 1626 | 1595 |
| 1606 | 1607 | 1557 |
| 1562 | 1562 | 1521 |
| 1521 | 1521 | 1495 |
| 1493 | 1492 | 1456 |
| 1469 | 1468 | 1422 |
| 1449 | 1449 | 1393 |
| 1431 | 1431 | 1371 |
| 1383 | 1400 | 1330 |
| 1377 | 1384 | 1285 |
| 1340 | 1312 | 1249 |
| 1312 | 1274 | 1223 |
| 1275 | 1236 | 1175 |
| 1231 | 1176 | 1164 |
| 1175 | 1159 | 1123 |
| 1156 | 1128 | 1099 |
| 1123 | 1096 | 1071 |
| 1095 | 1059 | 1049 |
| 1063 | 1023 | 1022 |
| 1024 | 1008 | 1008 |
| 1008 | 968 | 978 |

(continued)

| IR Bands [Peak maxima in cm-1] | | |
|---|---|---|
| **Modification A FIG. 10** | **Modification B FIG. 11** | **Modification C FIG. 12** |
| 967 | 944 | 961 |
| 908 | 920 | 921 |
| 848 | 907 | 907 |
| 834 | 848 | 862 |
| 829 | 833 | 837 |
| 812 | 820 | 814 |
| 783 | 813 | 775 |
| 763 | 783 | 765 |
| 738 | 764 | 753 |
| 717 | 738 | 733 |
| 666 | 724 | 725 |
| 656 | 683 | 678 |
| 636 | 661 | 665 |
| 609 | 656 | 659 |
| 599 | 636 | 633 |
| 591 | 621 | 593 |
| 570 | 613 | 581 |
| 556 | 600 | 570 |
| | 591 | 556 |
| | 570 | |
| | 557 | |

3.3 Raman bands obtained following the method as described under General Methods above

| Raman Bands [Peak maxima in $cm^{-1}$] | | |
|---|---|---|
| **Modification A FIG. 13** | **Modification B FIG. 14** | **Modification C FIG. 15** |
| 3079 | 3081 | 3076 |
| 2935 | 2942 | 3007 |
| 1693 | 1746 | 2952 |
| 1612 | 1696 | 2884 |
| 1495 | 1632 | 1708 |
| 1463 | 1610 | 1617 |
| 1448 | 1495 | 1606 |
| 1433 | 1464 | 1522 |
| 1340 | 1448 | 1469 |
| 1316 | 1432 | 1424 |

(continued)

| Raman Bands [Peak maxima in $cm^{-1}$] | | |
|---|---|---|
| **Modification A FIG. 13** | **Modification B FIG. 14** | **Modification C FIG. 15** |
| 1300 | 1339 | 1406 |
| 1286 | 1315 | 1372 |
| 1252 | 1300 | 1328 |
| 1180 | 1288 | 1298 |
| 1157 | 1271 | 1272 |
| 1107 | 1252 | 1224 |
| 1070 | 1180 | 1175 |
| 1009 | 1159 | 1164 |
| 955 | 1108 | 1130 |
| 849 | 1070 | 1095 |
| 815 | 1009 | 1072 |
| 784 | 843 | 1054 |
| 764 | 815 | 1019 |
| 735 | 784 | 1009 |
| 689 | 735 | 982 |
| 657 | 685 | 963 |
| 637 | 662 | 922 |
| 592 | 637 | 862 |
| 503 | 611 | 841 |
| 419 | 591 | 812 |
| 390 | 417 | 766 |
| 348 | 386 | 733 |
| 300 | 343 | 680 |
| 271 | 270 | 665 |
| 189 | 189 | 637 |
| 139 | 143 | 593 |
| | | 580 |
| | | 569 |
| | | 546 |
| | | 487 |
| | | 440 |
| | | 418 |
| | | 388 |
| | | 340 |
| | | 316 |
| | | 283 |
| | | 250 |

(continued)

| Raman Bands [Peak maxima in $cm^{-1}$] | | |
|---|---|---|
| **Modification A FIG. 13** | **Modification B FIG. 14** | **Modification C FIG. 15** |
| | | 165 |
| | | 141 |
| | | 120 |
| | | 92 |

3.4 DSC- / TGA-Thermograms

For the diagrams shown in the respective figures FIG. 16, 17, 18 the following devices and conditions were used, generally following the method as described under General Methods above:

| Figure | Mod | Device of Perkin Elmer | Heating for DSC // TGA | Heating rate for DSC/TGA | Sample Weight | Sample |
|---|---|---|---|---|---|---|
| FIG. 11 | C | PE-DSC-Diamond 2 | -10°C-160°C // 35°C-160°C | 20K/min // 10K/min | 3,839mg | Example 2.3) |
| FIG. 12 | B | PE-DSC-Diamond 2 | -10°C-160°C // 35°C-160°C | 20K/min // 10K/min | 3,076mg | Example 1.7 |
| FIG. 13 | A | DSC 8000 | -10°C-160°C // 35°C-160°C | 20K/min // 10K/min | 5,767mg | Example 2.1 |

For the TGA thermograms the method described under General Method is used for all probes.

**Example 4: Powder properties of modifications of (6S)-5-[4'-Fluoro-2-(trifluoromethyl)biphenyl-4-yl]-6-methyl-3,6-dihydro-2H-1,3,4-oxadiazin-2-one, [compound of formula (I) in a shear test**

The device used is the Schulze ring shear tester RST-XS.s for which a general technical product information is available on http://www.dietmar-schulze.de. We were using the shear cell XS-SV3. A statement referring to the testing procedure from the technical product information for RST-XS.s reads as follows:
"Testing procedure: The powder sample is contained in an annular shear cell "(FIG. 19B).

"A vertical load N] is applied through a thin loading rod on the annular lid. To shear the sample, the shear cell rotates relative to the lid (direction ω), and the torque necessary for shearing is determined from forces and F1 and F2 acting in a tie rod and a push rod. Following a well-defined procedure, which is executed computer-controlled, the flow properties are measured. All forces acting are transferred directly on the annular lid through thin rods. This ensures the accurate measurement of normal and shear forces without any friction forces having an influence on the test results."

For measuring flow properties of a wide range of bulk solids, the Schulze ring shear tester has proven its suitability in industrial practice. This device measurement is described in detail in: Schulze, D. (2008). Powders and bulk solids. Behaviour, Characterization, Storage and Flow. Springer Berlin Heidelberg New York ISBN 978-3-540-73767, chapter 4.1.2, p.84ff and comprises a shear cell having a volume of 3,5 ml, where the bulk solid is filled in, a lid closing the shear cell, a motor and guiding rolls.

FIG. 19A shows the Schulze ring shear tester as described in the book cited above.

Description of the numbers in Fig. 19A:

| | |
|---|---|
| *1* | ***Bottom ring*** |
| 2 | ***Lid of shear cell*** |
| 3 | ***Bulk solid*** |
| 4 | ***Tension rod*** |
| 5 | ***Crossbeam*** |

(continued)

| | |
|---|---|
| 6 | ***Guide roll*** |

Details of the shear cell as shown in FIG. 19B:

| **Typ** | **XS-SV3 (pyramides at bottom and lid)** |
|---|---|
| material | AlMgCuPb |
| **Cell:** | |
| $r_{ISZ}$ (fillet of the internal volume) | 12 mm = 1,2 cm |
| $r_{aSZ}$ (outer radius of the internal volume ) | 24 mm = 2,4 cm |
| $d_{ISZ}$ (inner diameter of the internal volume ) | 24 mm = 2,4 cm |
| $d_{aSZ}$ (external diameter of the internal volume ) | 48 mm = 4,8 cm |
| $h_{SZ}$ (nominal height of internal volume after filling) | 3 mm = 0,3 cm |
| $A_{SZ}$ (cross section area of sample room) | 13,57 $cm^2$ |
| $V_{SZ}$ ( internal volume, pyramids at bottom and lid not taken into account; $V_{SZ}=H_{SZ} \times A_{SZ}$) | 4,072 $cm^3$ |
| $V_{Mt}$ (Pyramides at bottom and lid) | 0,415 $cm^3$ |
| $h_{Mt}$ (effective height of pyramides at the lid) | 0,33 mm = 0,033 cm |
| **Lid:** | |
| $r_{ID}$ (inner radius of the annular lid) | 13 mm = 1,3 cm |
| $r_{aD}$ (external radius of the annular lid) | 23 mm = 2,3cm |
| $d_{ID}$ (inner diameter of the annular lid) | 26 mm = 2,6cm |
| $d_{aD}$ (external diameter of the annular lid) | 46 mm = 4,6 cm |
| $A_D$ (area of the annular lid) | 11,31 $cm^2$ |

Preparation:

In order to measure the flow properties, the bottom ring of the shear tester is filled with the bulk solid as obtained from example 1.6 (probe 1) or example 1.5 (no micronization, probe 2) to be characterized. The shear cell was fully filled with a sample of powder of the compound of formula (I) thereby preventing condensing the powder. Both samples, sample 1 and sample 2, were classified as non-hydroscopic applying the Pharmacopeia rules.

After carefully leveling the bulk solid's surface in the ring, the lid and crossbar were put onto the bottom ring. A hanger was connected to the middle of the crossbeam. By automatically putting weights onto the hanger, the bulk solids sample was subjected to a normal load N of 1,5kPa which induces a normal stress $\sigma$ in the sample. The force $F_A$, which was also applied to the middle of the crossbeam, compensates the weight of the crossbeam itself, as well as the weight of the lid and the hanger. At the ends of the cross beam, tension rods are positioned whose other ends are connected to load cells.

Test:

During the shear test, the bottom ring slowly rotated in the direction of the arrow with the low rotational speed $\omega$ of 2 rpm maximum . Rotation of the lid was prevented by the tension rods; the tensile forces $F_1$ and $F_2$ necessary to achieve this are measured by the load cells. From the normal and shear forces respectively stresses, the so-called yield locus was determined, which describes the flow behavior of a bulk solid. Major parameters of influence on flowability are stress state, particle size distribution (see its determination in example 5), temperature (24°C), humidity (low at 30%) and storage time (no storage time).

Results

The experimental data obtained were analyzed following the method described in Schulze, D. (2008). Powders and bulk solids. Behaviour, Characterization, Storage and Flow. Springer Berlin Heidelberg New York ISBN 978-3-540-73767 in order to obtain the graph illustrated by FIG. 20 Probe 1 is a sample of modification A and Probe 2 is a sample of modification C. FIG. 21 provides the flow factor of both samples as calculated on basis of the ring shear experiment. X-axis $\sigma$ [kPa] stands for the normal stress in the sample and the y-axis $\tau$[kPa] stands for shear stress. The dark blue squares show the pre-shear points and the red triangles show the shear points of the shear experiment of Probe 2. The red line is the yield locus of Probe 2. Analog to this the green squares are the pre-shear points and the light blue squares are the shear points of the shear experiment of Probe 1. The green line is the yield locus of Probe 1. The flow factor $ff_c$ can be derived from FIG. 20 as follows: Two Mohr circles can be constructed: the larger Mohr circle touches the yield locus and the pre-shear point is a Point of this circle (center point of the circle is on the x-axis). This circle represents the consolidation of the sample and the larger value of the crossing of the x-axis is called the consolidation stress $\sigma_1$. The second Mohr touches the yield locus and the origin is a point of this circle. The value where the circle crosses the x-axis is called the unconfined yield strength $\sigma_c$. The flow factor $ff_c$ can be calculated as ratio between the consolidation stress and the unconfined yield strength. Further details on the construction of the Mohr circles can be found in the book of D. Schulze cited above.

Both powders, of modification A (Probe 1) and C (Probe 2), show only a small difference in flow behaviour, yet modification C has a slight advantage, by flowing a bit better than modification A. The flow factors $ff_c$ as obtained are illustrated in FIG 21.

Furthermore, the bulk density could be measured as follows:

Determination of bulk density during shear tests

Because of the normal stress of 1,5 kPa applied on the top of the lid during the shear test the lid lowers into the shear cell and compresses the powder.

During the shear tests this lowering of the lid is measured and recorded. Since the volume of the bottom ring is known and weight of the sample measured before the beginning of the shear test the bulk density of the sample during the shear test is calculated

$$\rho_{b,shear} = \frac{m_{ges} - m_{cell}}{(h_c - h_l) \cdot A_{cell}}$$

With:

| | |
|---|---|
| $\rho_{b,shear}$: | Bulk density under shear |
| $m_{ges}$: | total mass of shear cell including powder sample (w/o lid) |
| $m_{cell}$: | net mass of shear cell (w/o powder sample, w/o lid) |
| $h_c$: | height of the bottom ring |
| $h_l$: | lowering of lid |
| $A_{cell}$: | bottom area of shear cell |

The calculation of the bulk density is done automatically with the evaluation software provided together with the shear tester.

The result is illustrated by FIG. 22, comparing p(shear)in [kg/m$^3$] of modification A (probe 1) and modification C (probe 2).

A significant difference between modification A (Probe 1) and C (Probe 2) could be observed: modification A,

represented by Probe 1 has only a bulk density of 270 kg/m$^3$ whereas modification C, represented by Probe 2, has a bulk density of about 550 kg/m$^3$. This results is an unexpected significant advantage of modification C in view of modification A regarding the storage volume, as in the same volume the double amount of substance of modification C can be stored.

**Example 5: Particle Size Distribution measured by Laser Diffraction of Modifications A and C of (6S)-5-[4'-Fluoro-2-(trifluoromethyl)biphenyl-4-yl]-6-methyl-3,6-dihydro-2H-1,3,4-oxadiazin-2-one, the compound of formula (I)**

The particle size distribution was measured by laser diffracation using the Malvern Panalytical Mastersizer 3000.
The samples are again taken from example 1.6 (probe 1) and example 1.5 (probe 2), the same as used in example 4.
The sample to be analyzed is dispersed in 1 drop of Tween 80 the sample is processed according to ISO13320.

Measurement of particle size distribution (PSD) by laser diffraction

Laser diffraction is an often-used method for determination the particle size of dispersed solid materials. A widened Laser beam illuminates a cuvette which contains part of the particles. By diffraction on the particles a characteristic pattern can be mapped on the detector. The resulting diffraction image is the additive overlaying of diffraction images of all particles.
Besides the mentioned detector two additional detectors are mounted in order to detect diffraction light in forward direction ($FA_{1..9}$), in wide angle ($LA_{1,2}$) and in back direction ($BS_{1,2}$). The turbidity of the sample is measured by an extinction sensor. This is necessary to ensure a low particle concentration which is necessary to avoid wrong results by multiple diffraction of light at several particles.
The cuvette used was a wet unit where the suspension is pumped in a closed circuit with a dispersing unit that ensures a separation of single particles by shear forces.
The measurement range of the Malvern 3000 covers a particle size from 1 mm (2 mm) down to 20 nm

Calculation

The calculation of the particle size distribution from the diffraction patterns was done on basis of theory of Fraunhofer.
Basis of the Fraunhofer Theory is that the signal of the detector is only induced by diffraction, that means that the particles are completely opaque and spherical. This assumption is valid for particle sizes of 10 to 50 μm and above. For smaller particles the light scattering of the particle itself due to optical transparency must be considered which is done in the Mie-theory (not used for our probes). Precondition of the application of the Mie-theory is the knowledge of the refractive and absorption index of the dispersed solids.

Resulting Figures

The results comparing particle size distributions between modification A and C are illustrated in FIG.23, wherein the y-axis shows the volume distribution Q3 [%] and the x-axis shows the particle size [μm], "ohne" means "without",
**[0171]** Q3 is the cumulative volume distribution, e.g. D50=Q(50%) indicates 50% of the volume part of the sample is smaller than particle size of 50μm.
The particle size is expressed as an equivalent diameter used in the method recommended by the Malvern Analysis Program and as described in ISO13320.
Information about the graphs as shown in FIG. 18 from left to the right:

- the first line (red line) relates to sample 1 (probe 1), modification A, with 300s ultrasound,
- the second line (blue line) relates to sample 2 (probe 2), modification C, with 300s ultrasound
- the third line (red dashed line) relates to sample 1 (probe 1), modification A, without ultrasound and
- the fourth line (blue dashed line) relates to sample 2 (probe 2), modification C, without ultrasound.

The particle size distribution is surprisingly significantly different between the two modifications, without ultrasound treatment modification A has a significantly broader particle size distribution and larger particles, compared to modification C whereas after ultrasound treatment modification A has a significantly smaller particle size and broader distribution than modification C. This broad distribution translates into possible segregation effects of modification A during transport and processing, whereas narrow distribution of modification C reduces segregation effects. Also, a large influence of ultrasound treatment during measurement of modification A might indicate undesired fragility of particles

compared to modification C.

| **Sample name** | **Probe 1** | **Probe 2** | **Probe 1** | **Probe2** |
|---|---|---|---|---|
| **Remark** | without Ultrasound (US) | without US | 300s US | 300s US |
| **D(10)** | 165 | 69,1 | 4,02 | 14,5 |
| **D(50)** | 404 | 179 | 44 | 57,3 |
| **D(90)** | 720 | 380 | 128 | 170 |

Dx=particle size, e.g. D(10)=particle size at Q3=10% is 165 means that 10% of the volume part of the sample is smaller than particle size of 165 μm.

The data of the following table are the basis for FIG. 23, showing for 4 probes for certain **particle sizes D*[μm]*** *(colmn* 1) the **Cumulative Distribution Q3(x)** [%] in columns 2-5, whereby the conditions used for the probes are indicated as "without ultrasound (=US)" or "300 s US":

| ***Probe name*** | **Probe 1** | **Probe 2** | **Probe 1** | **Porbe2** |
|---|---|---|---|---|
| ***Laser obscuration*** | 12,18 | 9,44 | 23,63 | 22,79 |
| | | | | |
| ***Particle size D [μm] / Cumulative volume distribution Q [%], Remark for condition*** | without US | without US | 300s US | 300s US |
| *0,01* | 0 | 0 | 0 | 0 |
| *0,0114* | 0 | 0 | 0 | 0 |
| *0,0129* | 0 | 0 | 0 | 0 |
| *0,0147* | 0 | 0 | 0 | 0 |
| *0,0167* | 0 | 0 | 0 | 0 |
| *0,0189* | 0 | 0 | 0 | 0 |
| *0,0215* | 0 | 0 | 0 | 0 |
| *0,0244* | 0 | 0 | 0 | 0 |
| *0,0278* | 0 | 0 | 0 | 0 |
| *0,0315* | 0 | 0 | 0 | 0 |
| *0,0358* | 0 | 0 | 0 | 0 |
| *0,0407* | 0 | 0 | 0 | 0 |
| *0,0463* | 0 | 0 | 0 | 0 |
| *0,0526* | 0 | 0 | 0 | 0 |
| *0,0597* | 0 | 0 | 0 | 0 |
| *0,0679* | 0 | 0 | 0 | 0 |
| *0,0771* | 0 | 0 | 0 | 0 |
| *0,0876* | 0 | 0 | 0 | 0 |
| *0,0995* | 0 | 0 | 0 | 0 |
| *0,113* | 0 | 0 | 0 | 0 |
| *0,128* | 0 | 0 | 0 | 0 |
| *0,146* | 0 | 0 | 0 | 0 |
| *0,166* | 0 | 0 | 0 | 0 |
| *0,188* | 0 | 0 | 0 | 0 |

(continued)

| *Probe name* | Probe 1 | Probe 2 | Probe 1 | Porbe2 |
|---|---|---|---|---|
| *0,214* | 0 | 0 | 0 | 0 |
| *0,243* | 0 | 0 | 0 | 0 |
| *0,276* | 0 | 0 | 0 | 0 |
| *0,314* | 0 | 0 | 0 | 0 |
| *0,357* | 0 | 0 | 0,06 | 0 |
| *0,405* | 0 | 0 | 0,15 | 0 |
| *0,46* | 0 | 0 | 0,27 | 0 |
| *0,523* | 0 | 0 | 0,42 | 0 |
| *0,594* | 0 | 0 | 0,62 | 0,08 |
| *0,675* | 0 | 0 | 0,85 | 0,2 |
| *0,767* | 0 | 0 | 1,14 | 0,33 |
| *0,872* | 0 | 0 | 1,46 | 0,5 |
| *0,991* | 0 | 0 | 1,84 | 0,67 |
| *1,13* | 0 | 0 | 2,26 | 0,86 |
| *1,28* | 0 | 0 | 2,74 | 1,04 |
| *1,45* | 0 | 0 | 3,26 | 1,21 |
| *1,65* | 0,07 | 0 | 3,84 | 1,37 |
| *1,88* | 0,14 | 0 | 4,48 | 1,52 |
| *2,13* | 0,23 | 0 | 5,18 | 1,67 |
| *2,42* | 0,32 | 0 | 5,97 | 1,83 |
| *2,75* | 0,43 | 0 | 6,84 | 2,02 |
| *3,12* | 0,56 | 0 | 7,8 | 2,24 |
| *3,55* | 0,69 | 0 | 8,87 | 2,49 |
| *4,03* | 0,84 | 0 | 10,03 | 2,8 |
| *4,58* | 1,01 | 0 | 11,28 | 3,16 |
| *5,21* | 1,18 | 0 | 12,61 | 3,57 |
| *5,92* | 1,37 | 0 | 14 | 4,05 |
| *6,72* | 1,56 | 0,06 | 15,43 | 4,6 |
| *7,64* | 1,76 | 0,13 | 16,9 | 5,22 |
| *8,68* | 1,97 | 0,2 | 18,38 | 5,91 |
| *9,86* | 2,17 | 0,27 | 19,88 | 6,7 |
| *11,2* | 2,38 | 0,35 | 21,41 | 7,61 |
| *12,7* | 2,6 | 0,43 | 22,99 | 8,67 |
| *14,5* | 2,81 | 0,52 | 24,65 | 9,93 |
| *16,4* | 3,03 | 0,66 | 26,43 | 11,44 |
| *18,7* | 3,24 | 0,85 | 28,38 | 13,29 |
| *21,2* | 3,45 | 1,12 | 30,55 | 15,54 |
| *24,1* | 3,66 | 1,51 | 33,01 | 18,26 |

(continued)

| *Probe name* | Probe 1 | Probe 2 | Probe 1 | Porbe2 |
|---|---|---|---|---|
| *27,4* | 3,86 | 2,02 | 35,82 | 21,51 |
| *31,1* | 4,09 | 2,66 | 39,04 | 25,33 |
| *35,3* | 4,33 | 3,42 | 42,69 | 29,7 |
| *40,1* | 4,62 | 4,3 | 46,79 | 34,58 |
| *45,6* | 4,97 | 5,3 | 51,31 | 39,87 |
| *51,8* | 5,38 | 6,44 | 56,19 | 45,48 |
| *58,9* | 5,84 | 7,78 | 61,32 | 51,26 |
| *66,9* | 6,34 | 9,44 | 66,6 | 57,08 |
| *76* | 6,84 | 11,6 | 71,86 | 62,81 |
| *86,4* | 7,31 | 14,47 | 76,95 | 68,33 |
| *98,1* | 7,71 | 18,26 | 81,73 | 73,52 |
| *111* | 8,06 | 23,13 | 86,06 | 78,28 |
| *127* | 8,42 | 29,12 | 89,81 | 82,52 |
| *144* | 8,94 | 36,15 | 92,92 | 86,16 |
| *163* | 9,85 | 43,99 | 95,36 | 89,2 |
| *186* | 11,47 | 52,3 | 97,15 | 91,66 |
| *211* | 14,12 | 60,66 | 98,37 | 93,62 |
| *240* | 18,13 | 68,67 | 99,12 | 95,18 |
| *272* | 23,71 | 75,96 | 99,54 | 96,46 |
| *310* | 30,88 | 82,27 | 99,76 | 97,5 |
| *352* | 39,48 | 87,49 | 99,87 | 98,37 |
| *400* | 49,13 | 91,58 | 99,94 | 99,05 |
| *454* | 59,27 | 94,65 | 100 | 99,55 |
| *516* | 69,25 | 96,82 | 100 | 99,86 |
| *586* | 78,43 | 98,26 | 100 | 100 |
| *666* | 86,25 | 99,16 | 100 | 100 |
| *756* | 92,34 | 99,65 | 100 | 100 |
| *859* | 96,59 | 99,9 | 100 | 100 |
| *976* | 99,14 | 100 | 100 | 100 |
| *1110* | 100 | 100 | 100 | 100 |
| *1260* | 100 | 100 | 100 | 100 |
| *1430* | 100 | 100 | 100 | 100 |
| *1630* | 100 | 100 | 100 | 100 |
| *1850* | 100 | 100 | 100 | 100 |
| *2100* | 100 | 100 | 100 | 100 |
| *2390* | 100 | 100 | 100 | 100 |
| *2710* | 100 | 100 | 100 | 100 |
| *3080* | 100 | 100 | 100 | 100 |

(continued)

| *Probe name* | Probe 1 | Probe 2 | Probe 1 | Porbe2 |
|---|---|---|---|---|
| *3500* | 100 | 100 | 100 | 100 |

Figure 24A shows particle size distributions of the sample of modification A without ultrasound treatment (measured three times) and after different times of ultrasound treatment. The green curves in the right side of the diagram are reflecting the measurement without ultrasound treatment and almost lie one above the other whereas curves reflecting the particle size distribution depending on the ultrasound treatment time, 15 s, 60 s, 180 s and 300 s, results in different curves showing the volume distribution moving from the right to the left, also indicated by D(50) becoming smaller and smaller when the treatment time became longer

Figure 24B shows particle size distribution of modification C without ultrasound treatment (measured three times) and after different times of ultrasound treatment. The green curves reflecting the measurement without ultrasound treatment almost lie one above the other whereas curves reflecting the particle size distribution depending on the ultrasound treatment time, 15 s, 60 s, 180 s and 300 s, results in different curves showing the volume distribution moving from the right to the left, also indicated by D(50) becoming smaller and smaller when the treatment time became longer.

The difference between the Q3[50] of the untreated samples and the sample treated 300s with ultrasound is different for modification A and modification C. Whereas for modification A the difference is large the difference for modification C is significantly smaller.

Furthermore the amount of fines after US treatment of 300 s is significantly larger for modification A than for modification C indicated by a much smaller values of D(10 %), D(20 %) and D(30 %).

Altogether this indicates that the crystals of modification C are more robust than those of modification A translating into less abrasion during any processing and hence loss of material which is a significant advantage for handling the modification e.g. for weighting, granulation, transport through pipes, to fill up the active ingredient for transport, for preparation of a composition for treatment.

## Claims

1. A crystalline form C of the compound of the formula (I)

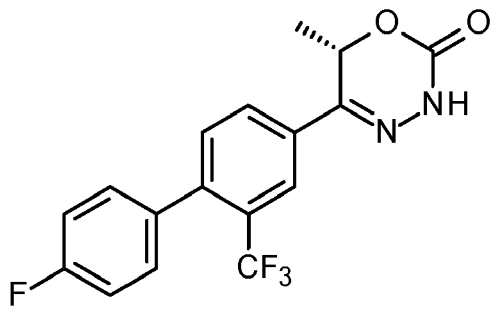

(I)

which is the modification C.

2. The form of the compound of claim 1 **characterized by** a X-ray powder diffractogram measured at 25°C and with Cu-K alpha 1 as radiation source displaying at least the following reflections, quoted as 2Θ value ± 0.2°: 21,1; 15,1; 22,6 ].

3. The form of the compound of claim 1 **characterized by** a X-ray powder diffractogram measured at 25°C and with Cu-K alpha 1 as radiation source displaying at least the following reflections, quoted as 2Θ value ± 0.2°: 21,1; 15,1; 22,6; 18,8; 26,8;.

4. The form of the compound of claim 1 **characterized by** a X-ray powder diffractogram measured at 25°C and with Cu-K alpha 1 as radiation source displaying at least the following reflections, quoted as 2Θ value ± 0.2°: 21,1; 15,1;

22,6; 18,8; 26,8; 22,6; 18,9;.

5. The form of the compound of claim 1 **characterized by** a X-ray powder diffractogram measured at 25°C and with Cu-K alpha 1 as radiation source displaying at least the following reflections, quoted as 2Θ value ± 0.2°: 21,1; 15,1; 22,6; 18,8; 26,8; 22,6; 18,9; 29,8; 11,9; 31,6;

6. The form C of the compound of formula (I) **characterized by** Raman spectroscopy measured at 25°C displaying at least the following reflections: 1606, 1617, 141 [cm-1]

7. The form C of the compound of formula (I) **characterized by** Raman spectroscopy measured at 25°C displaying at least the following reflections: 1606, 1617, 141, 92, 1272 [$cm^{-1}$].

8. The form C of the compound of formula (I) **characterized by** Raman spectroscopy measured at 25°C displaying at least the following reflections: 1606, 1617, 141, 92, 1272, 120, 1328 [cm-1].

9. The form C of the compound of formula (I) **characterized by** Raman spectroscopy measured at 25°C displaying at least the following reflections: 1606, 1617, 141, 92, 1272, 120, 1328, 812, 1095, 1298 [cm-1]

10. A pharmaceutical composition comprising modification C of the compound of formula (I) and optionally further pharmaceutically acceptable excipients.

11. The pharmaceutical composition of claim 10 comprising modification C of the compound of the formula (I) mainly and no significant fractions of another form of the compound of formula (I) and optionally further pharmaceutically acceptable excipients

12. The pharmaceutical composition according to claim 10 comprising modification C of the compound of formula (I) mainly and less than 15 % of another form or other forms of the compound of formula (I) and optionally further pharmaceutically acceptable excipients.

13. The pharmaceutical composition according to claim 12, wherein the amount of another form or other forms of the compound of formula (I) is less than 10 %.

14. The pharmaceutical composition according to claim 12, wherein the amount of another form or other forms of the compound of formula (I) is less than 5 %.

15. A crystalline form of the compound of any of claims 1 to 9 for use in the treatment and/or prophylaxis of diseases.

16. The crystalline form for use according to claim 15, whereby the disease is a hyperproliferative diseases.

17. The crystalline form for use according to claim 16, whereby the hyperproliferative disease is a cancer disease.

18. The crystalline form for use according to claim 17, whereby the cancer disease is selected from brain cancer, breast cancer, cervical cancer, head and neck cancer, leukemia, AML, lung cancer, lymphoma, skin cancer, esophagal carcinoma, ovarian cancer, pancreas cancer, prostate cancer and sarcoma.

19. The pharmaceutical composition of claim 10 or 11 for use in the treatment of a hyperproliferative disease.

20. The pharmaceutical composition for use according to claim 19, whereby the hyperproliferative disease is a cancer disease.

21. The pharmaceutical composition for use according to claim 19, whereby the cancer disease is selected from brain cancer, breast cancer, cervical cancer, head and neck cancer, leukemia, AML, lung cancer, lymphoma, skin cancer, esophagal carcinoma, ovarian cancer, pancreas cancer, prostate cancer and sarcoma.

22. The crystalline form for use as defined in any of claim 15-17 for the manufacture of a pharmaceutical composition for the treatment or prevention of brain cancer, breast cancer, cervical cancer, head and neck cancer, leukemia, AML, lung cancer, lymphoma, skin cancer, esophagal carcinoma, ovarian cancer, pancreas cancer, prostate cancer and sarcoma.

**23.** Use of a compound as defined in any of claims 1 to 9 for the manufacture of a pharmaceutical composition for the treatment or prevention of a cancer disease.

**24.** Use of a compound as defined in any of claims 1 to 6 for the manufacture of a stable pharmaceutical composition.

**25.** A method of treating or preventing of hyperproliferative diseases in a mammal, comprising administering to a mammal in need thereof a therapeutically effective amount of modification C of the compound of formula (I) according to claim 1.

FIG 1: XRPD modification C according to example 1.5

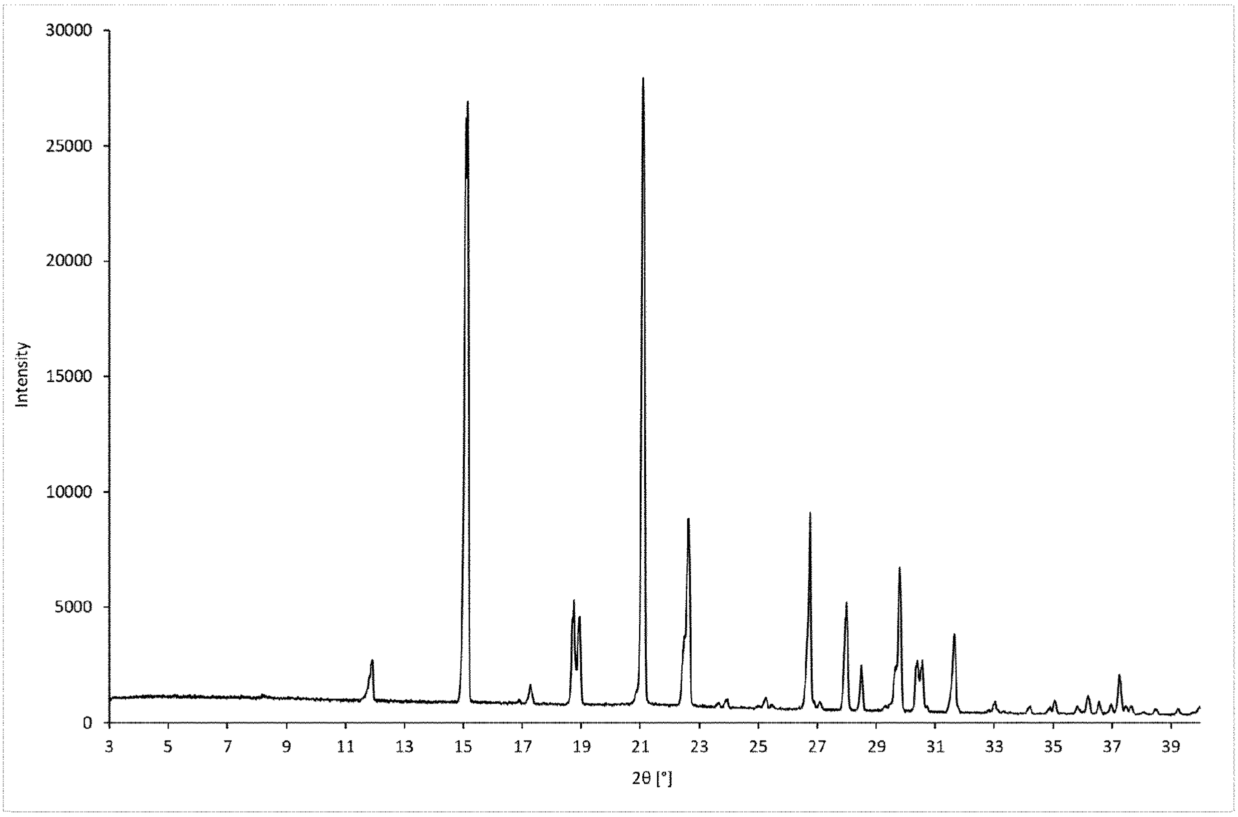


FIG. 1

FIG 2: XRPD modification C according to example 1.5, repeated batch

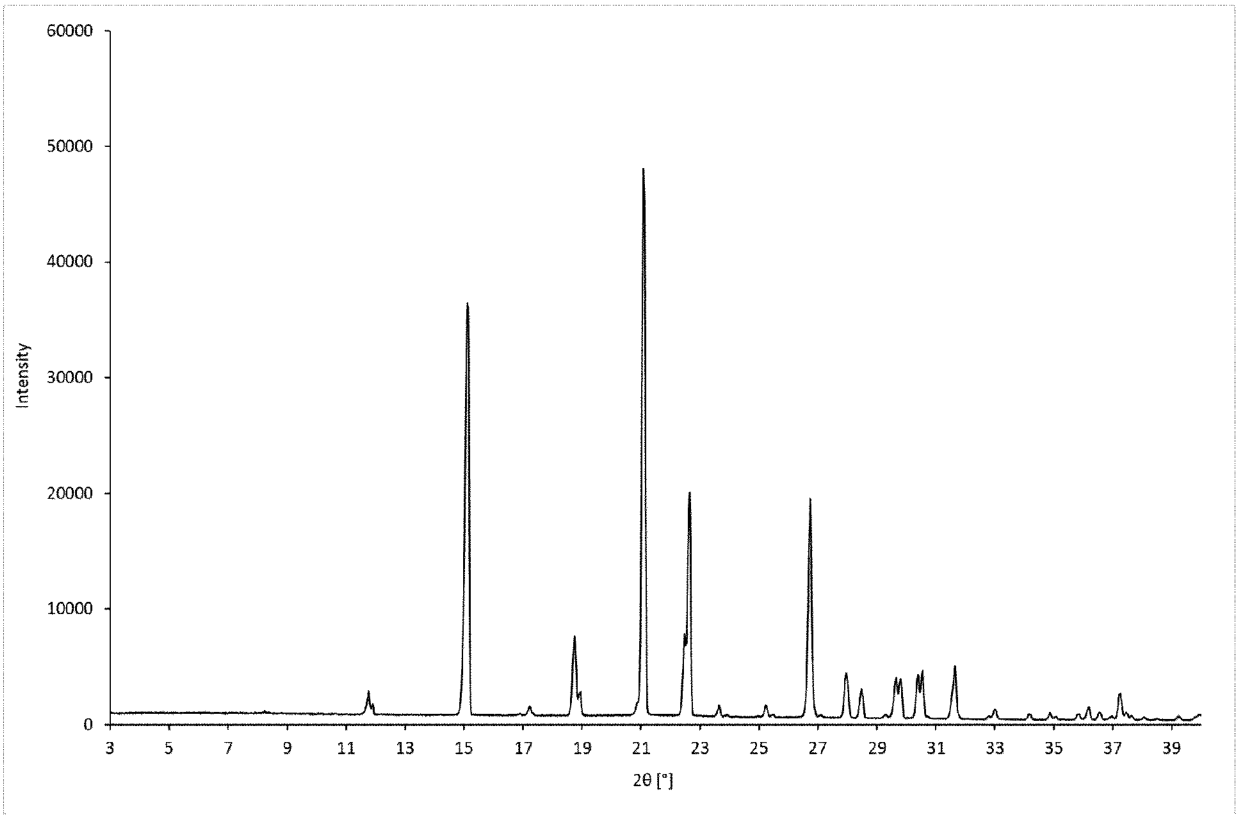


FIG. 2

FIG 3: XRPD modification A according to example 1.6

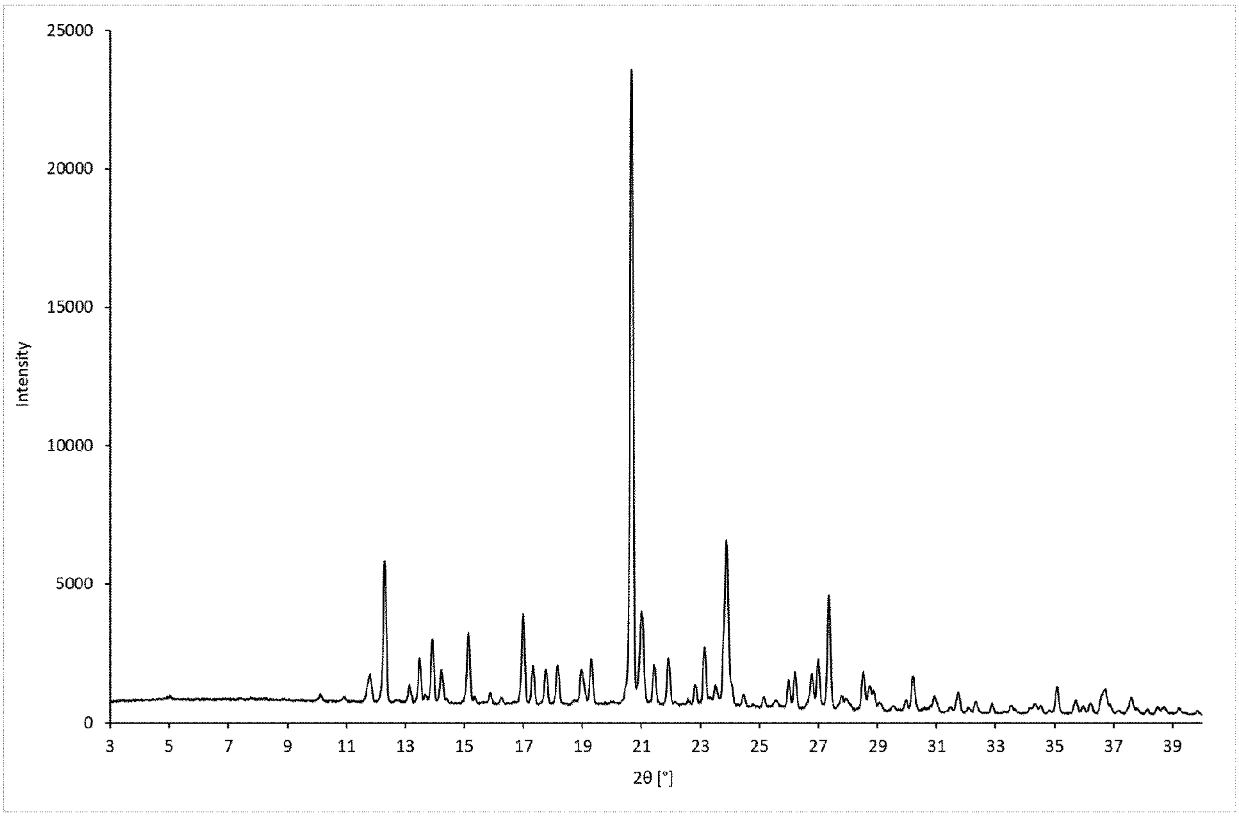


FIG. 3

FIG 4: XRPD modification B according to example 1.7

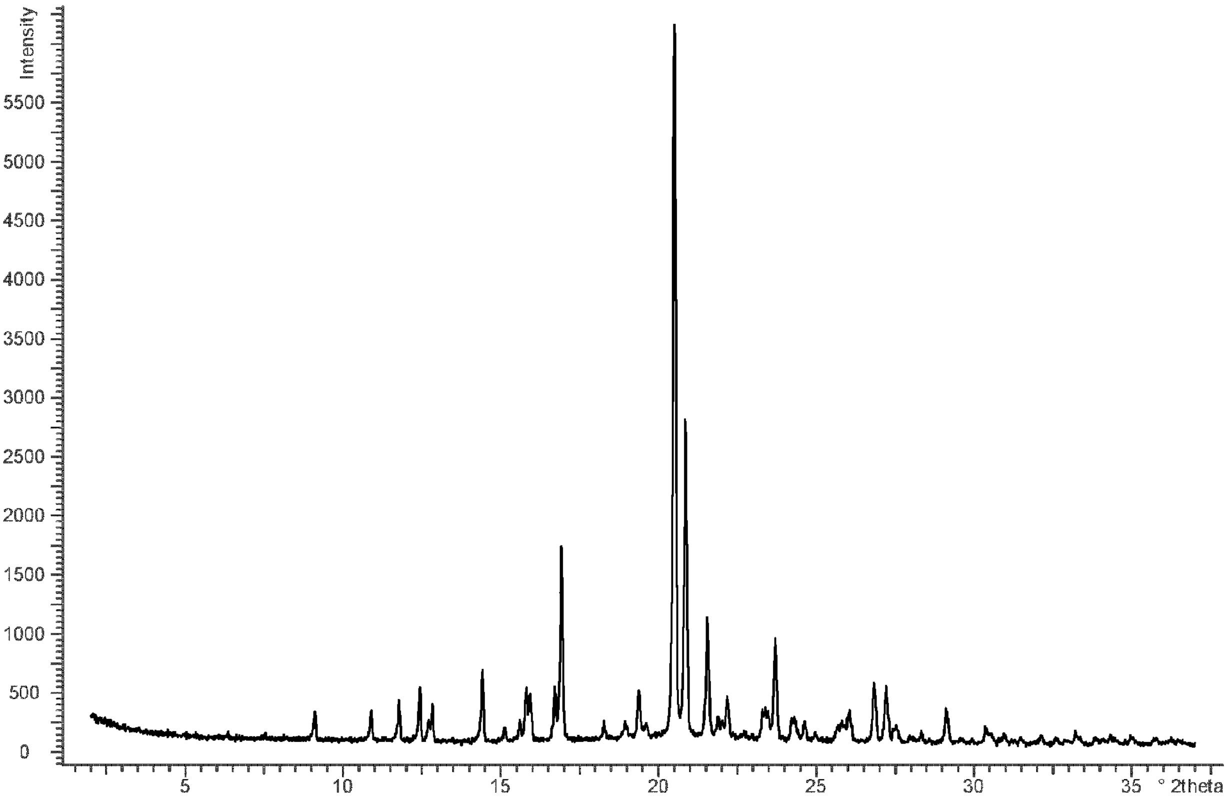


FIG. 4

FIG 5: XRPD modification B according to example 1.8, 1)

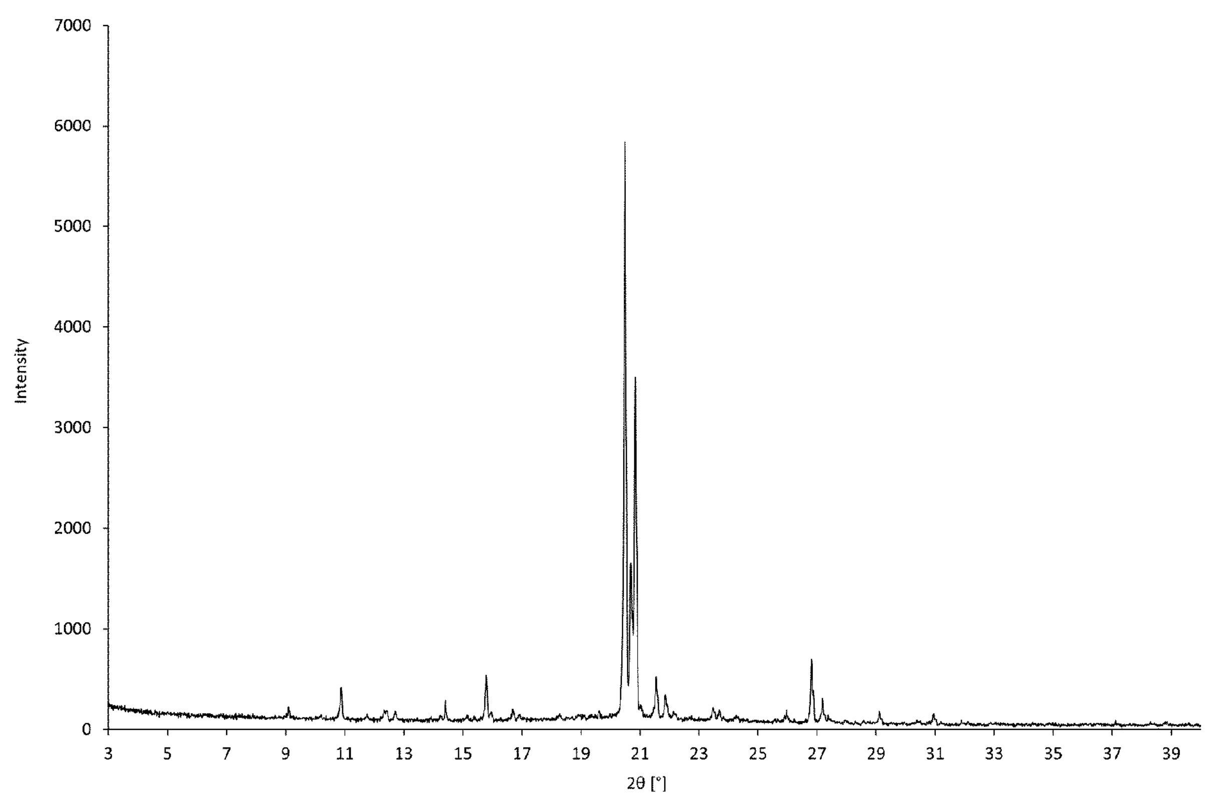


FIG. 5

FIG 6: XRPD modification C according to example 1.8, 2)

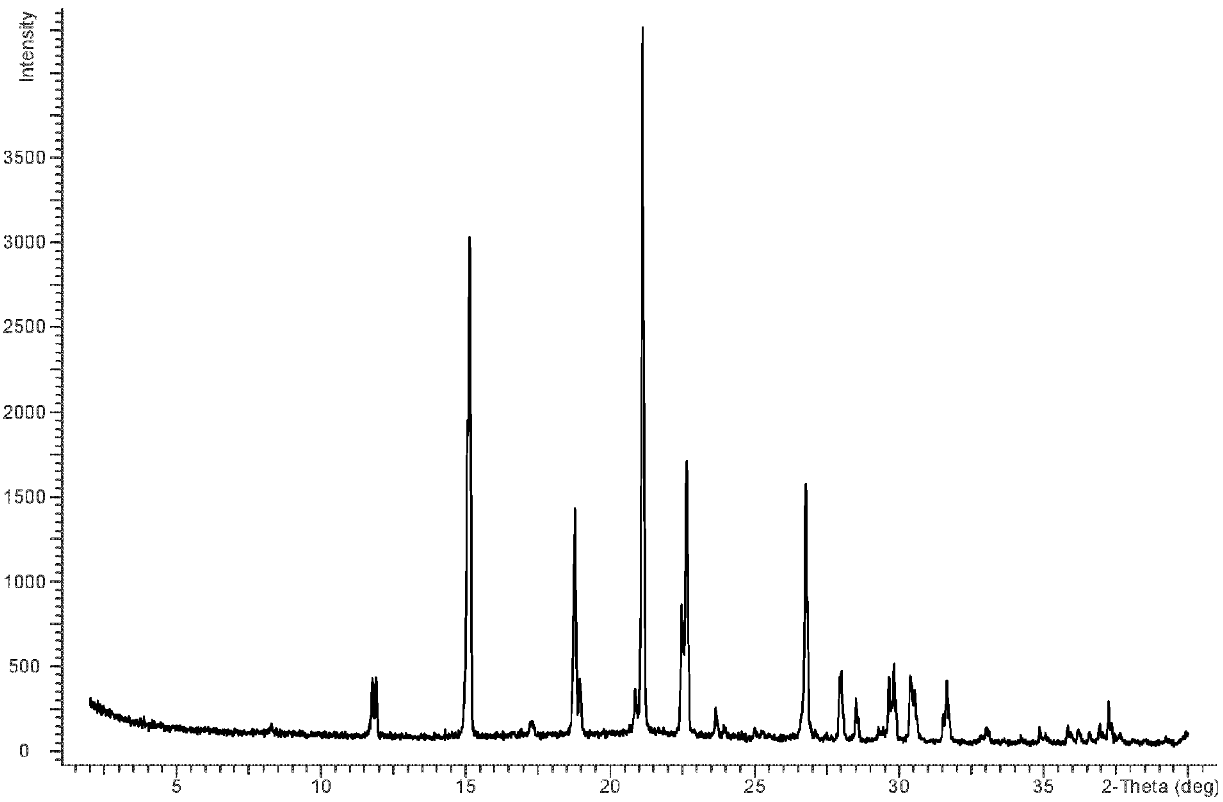


FIG. 6

FIG 7: XRPD modification A according to example 2.1/table in example 3.1

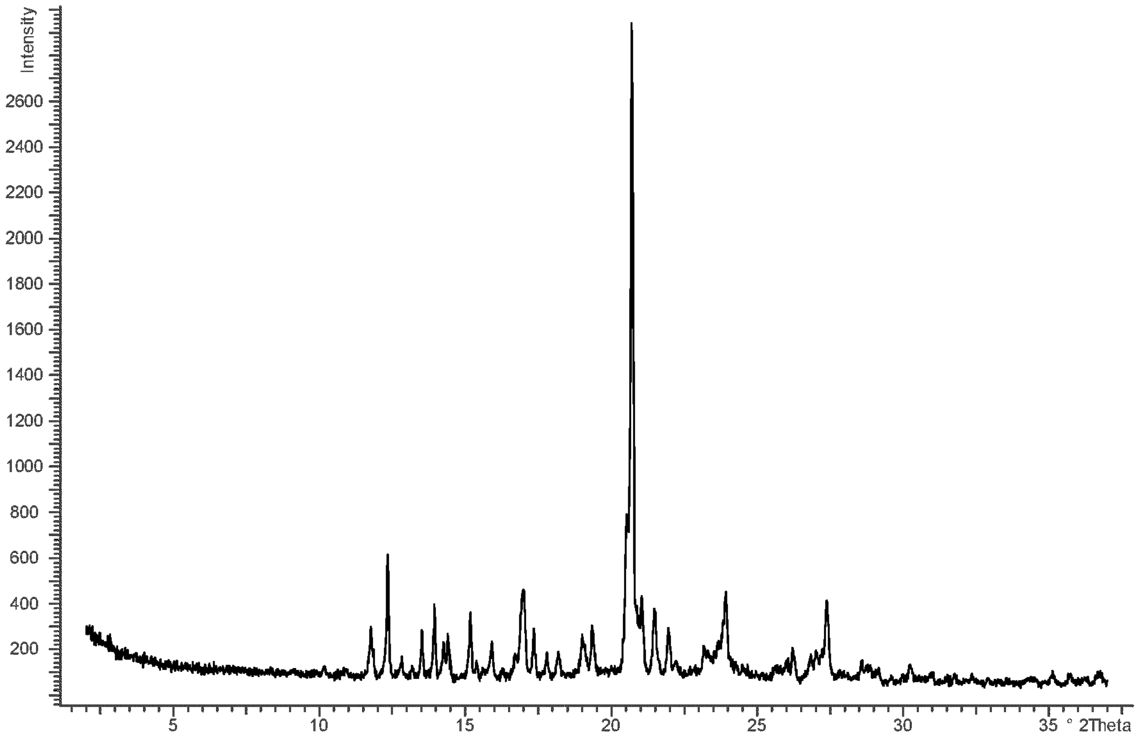


FIG. 7

FIG 8: XRPD modification B according to example 2.2/table in example 3.1

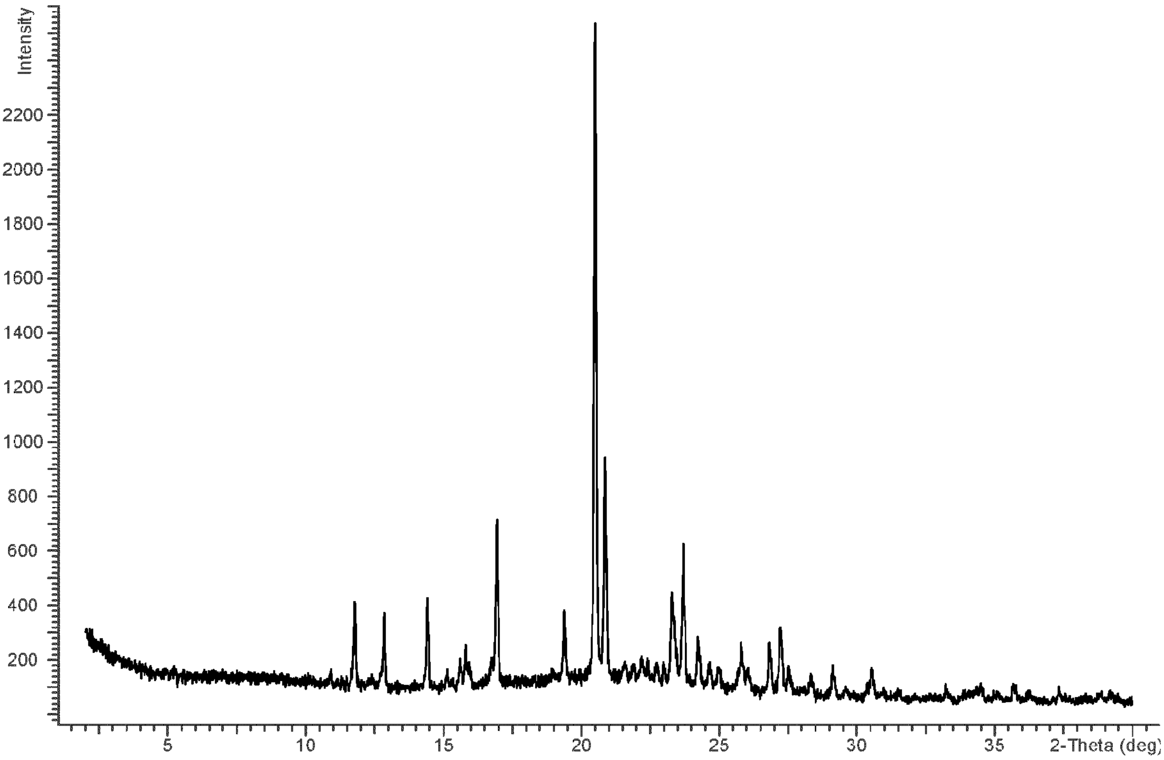


FIG. 8

FIG 9: XRPD modification C according to example 2.3/ table in example 3.1

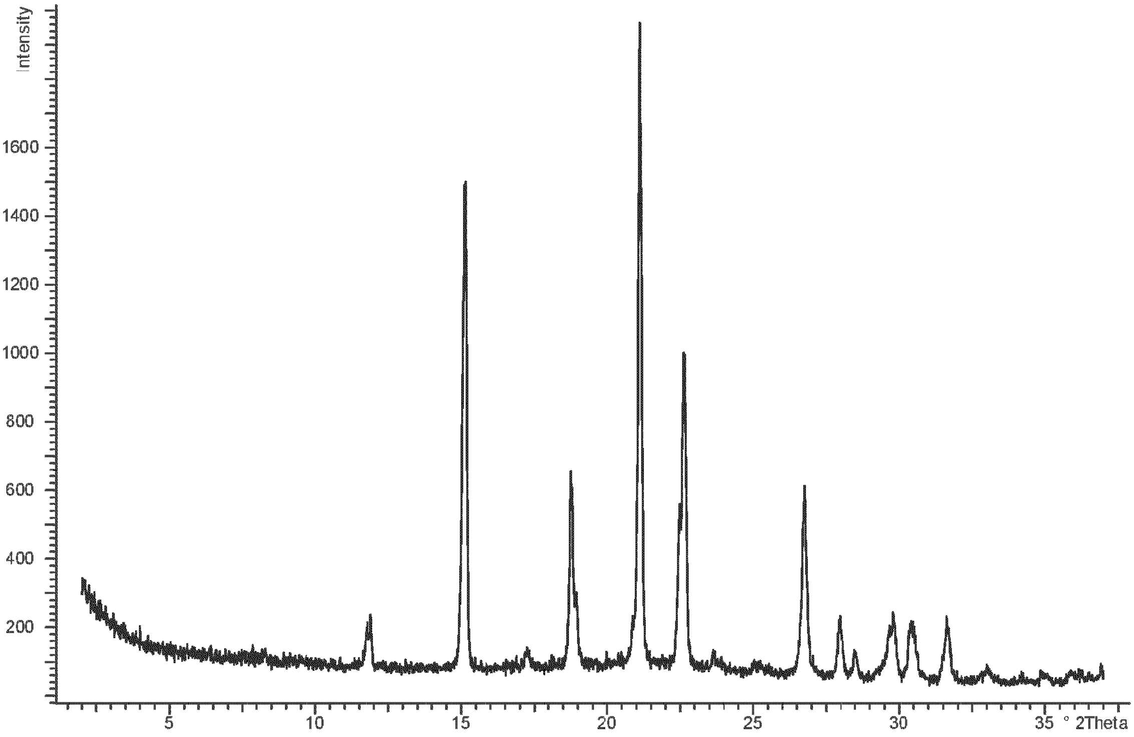


FIG. 9

FIG 10: IR spectrum of modification A according to example 2.1/ table in example 3.2

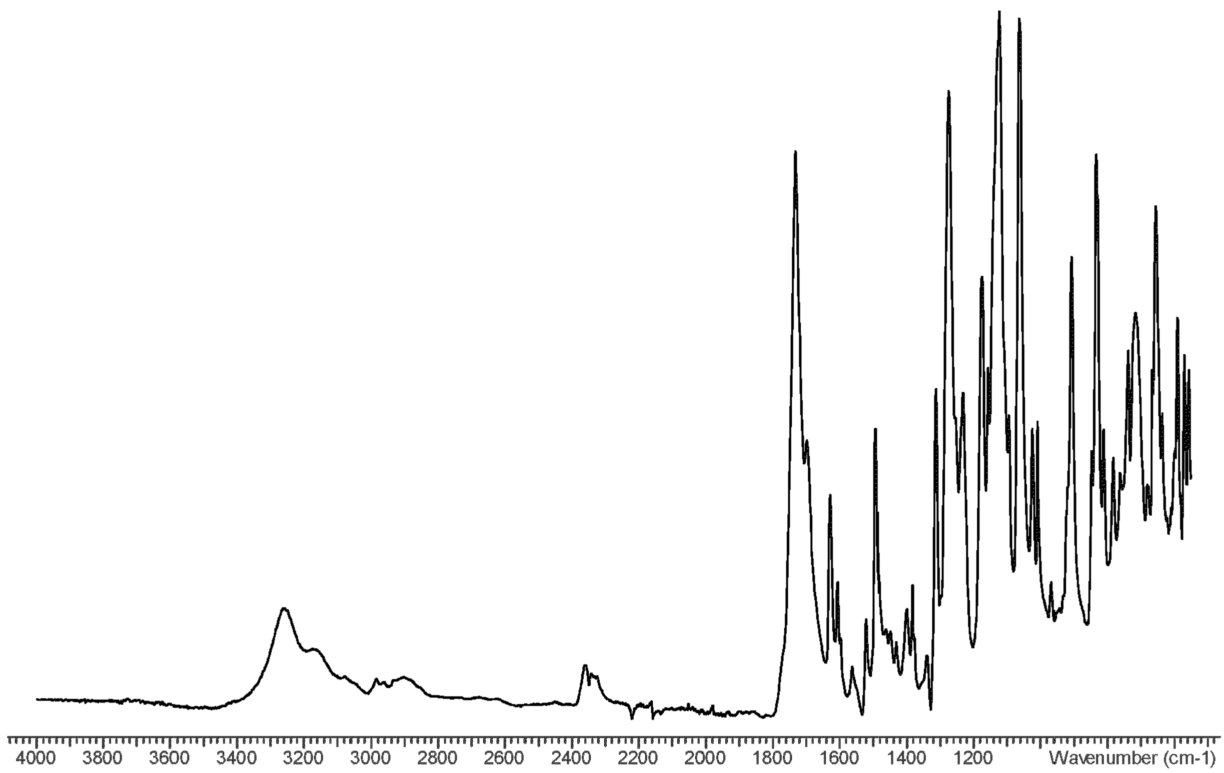


FIG. 10

FIG 11: IR spectrum of modification B according to example 2.2 / table in example 3.2

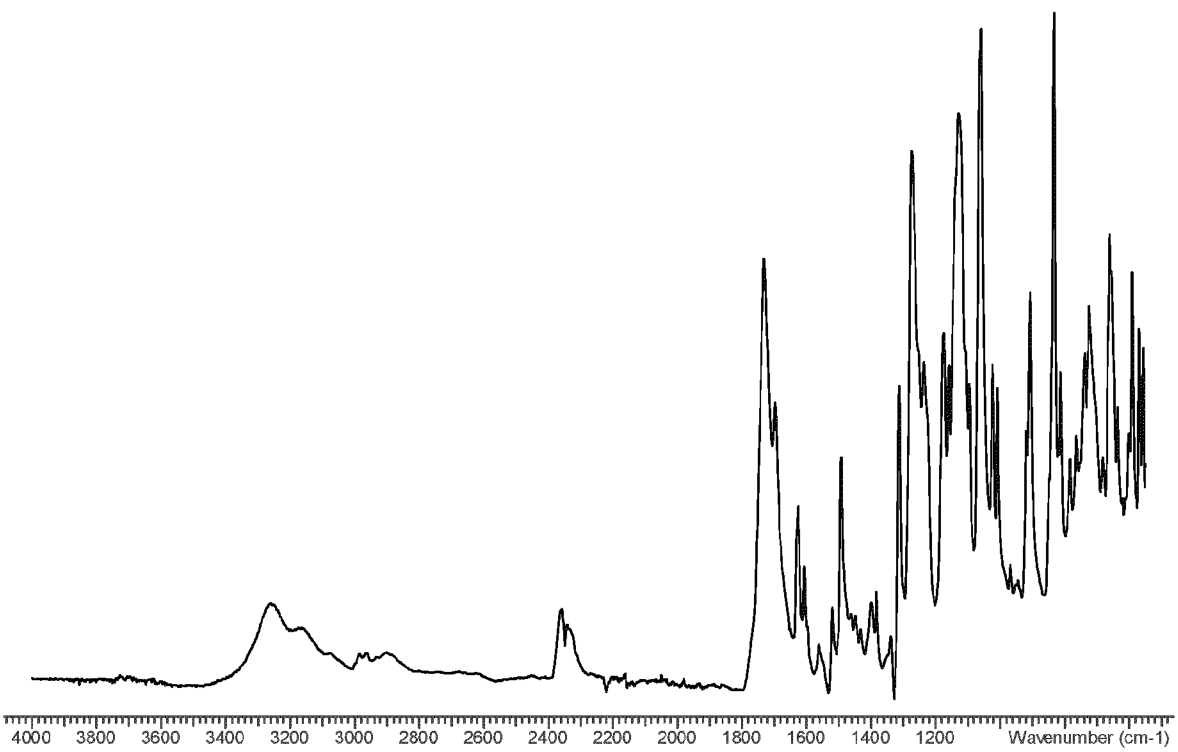


FIG. 11

FIG 12: IR spectrum of modification C according to example 2.3 / table in example 3.2

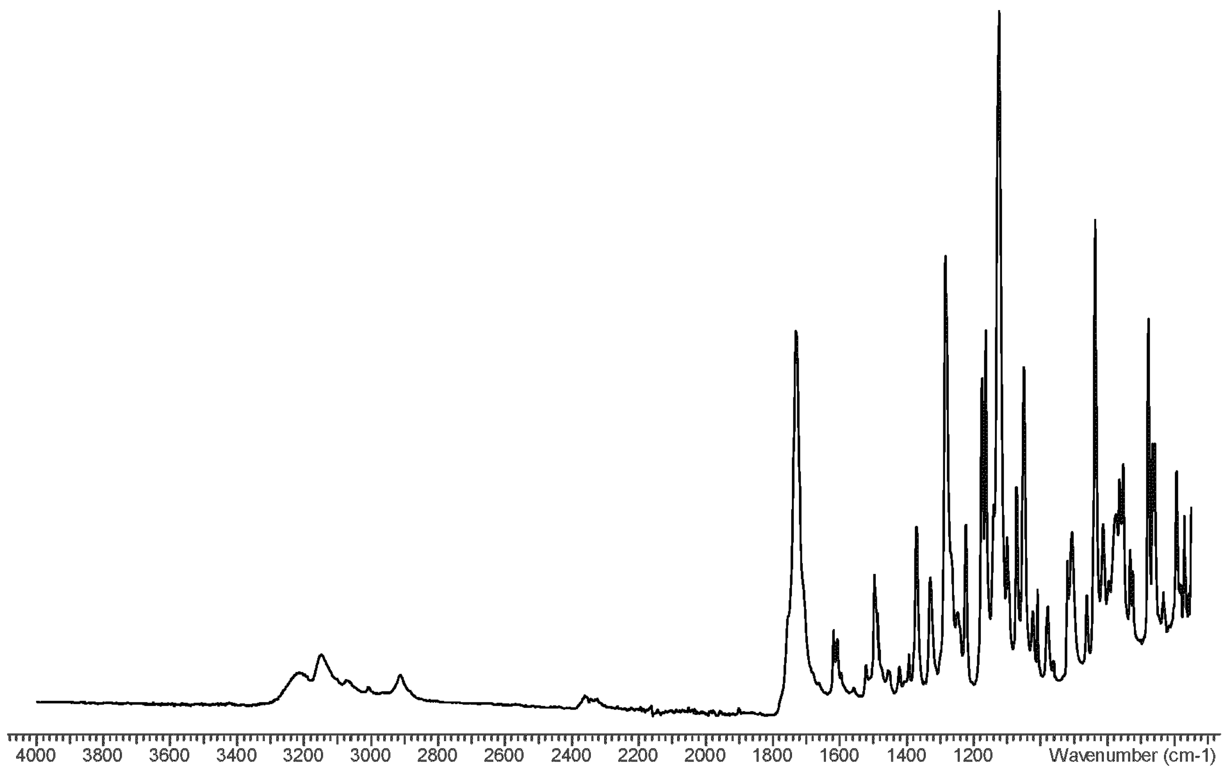


FIG. 12

FIG 13: Raman spectrum of modification A according to example 2.1 / table in example 3.3

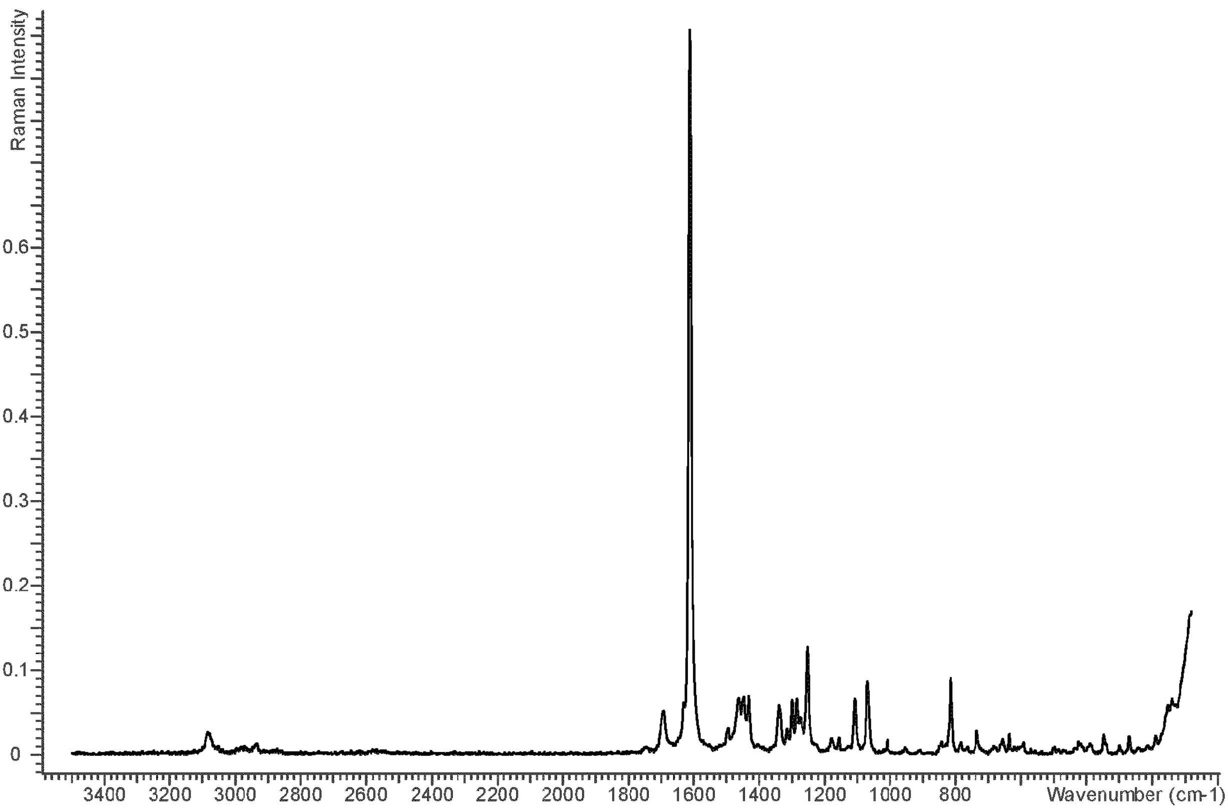


FIG. 13

FIG 14: Raman spectrum of modification B according to example 2.2 / table in example 3.3

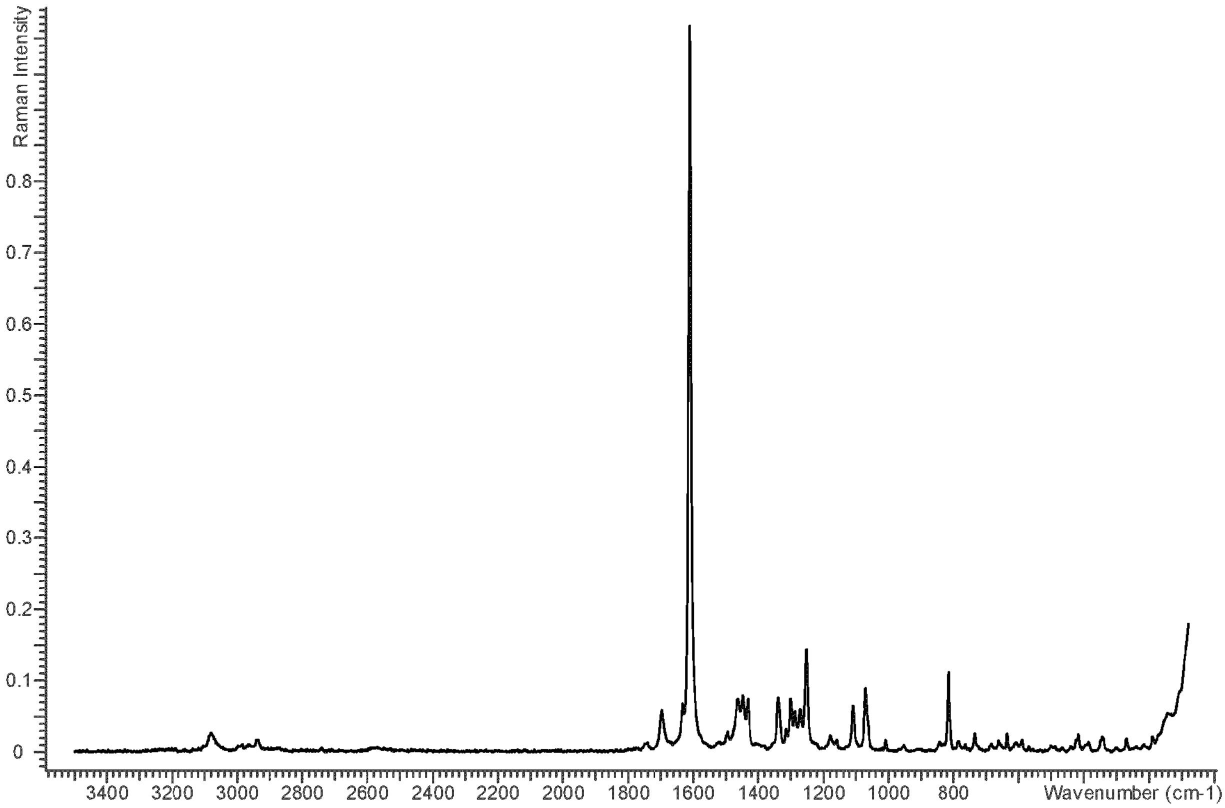


FIG. 14

FIG 15: Raman spectrum of modification C according to example 2.3 / table in example 3.3

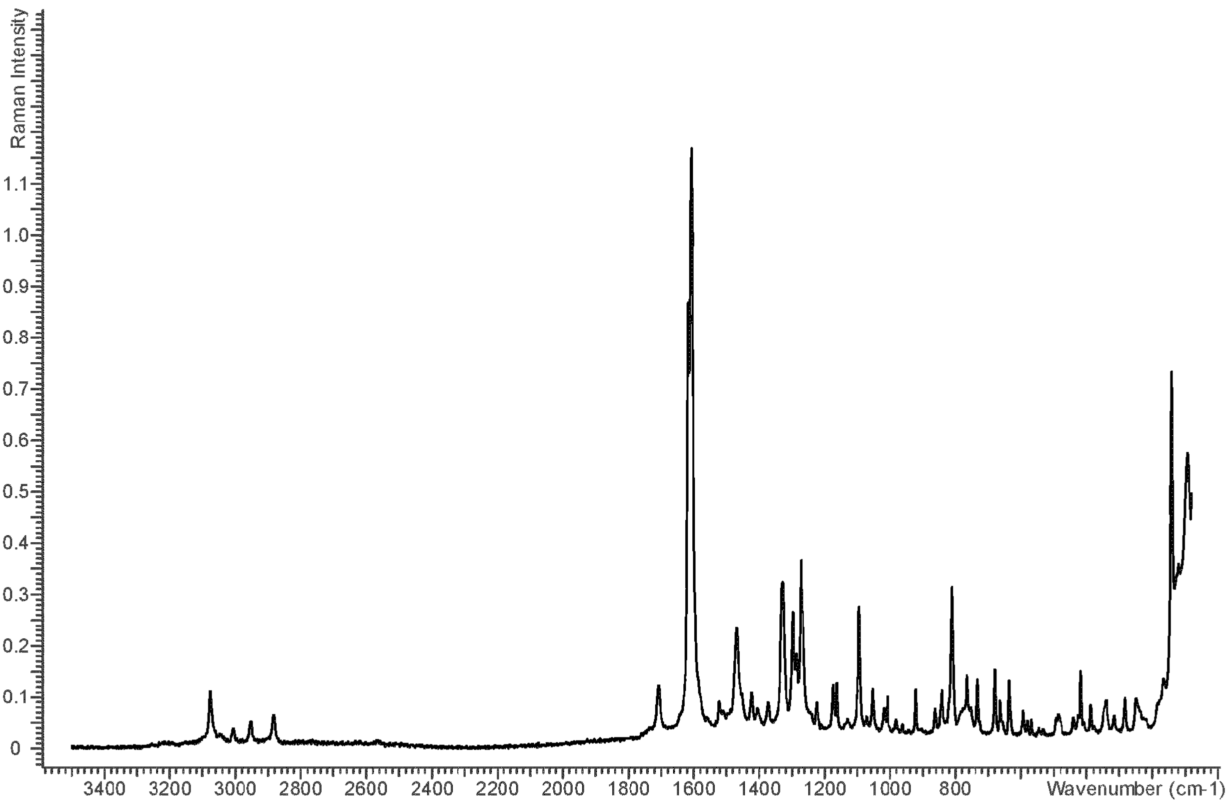


FIG. 15

FIG 16: DSC- and TGA-Thermogram of modification A according to example 2.1

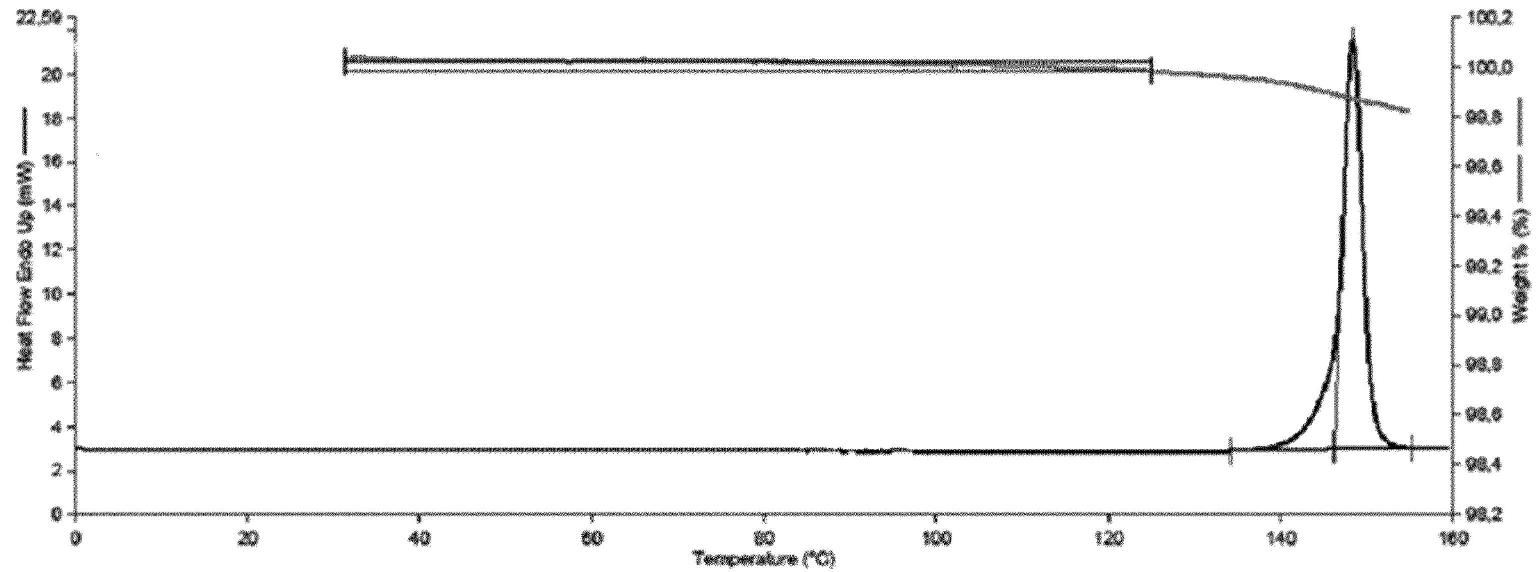


FIG.16

FIG 17: DSC- and TGA-Thermogram of modification B according to example 2.2

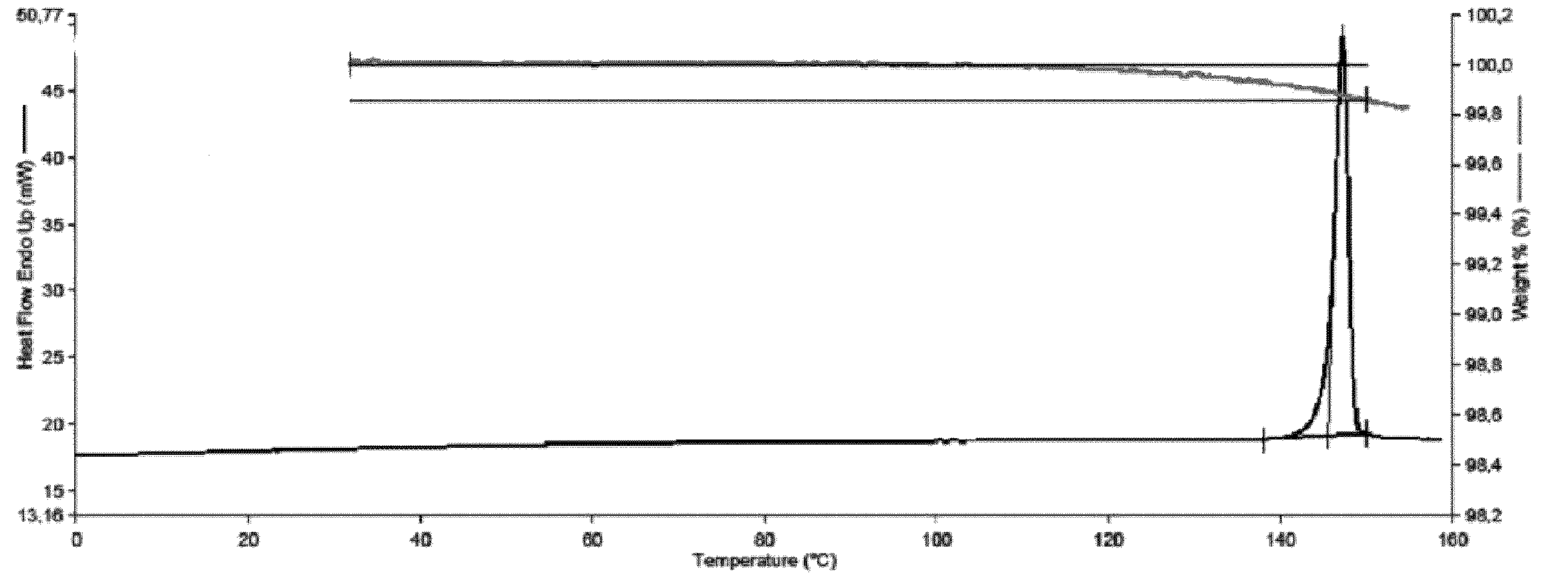


FIG.17

FIG 18: DSC- and TGA-Thermogram of modification C according to example 2.3

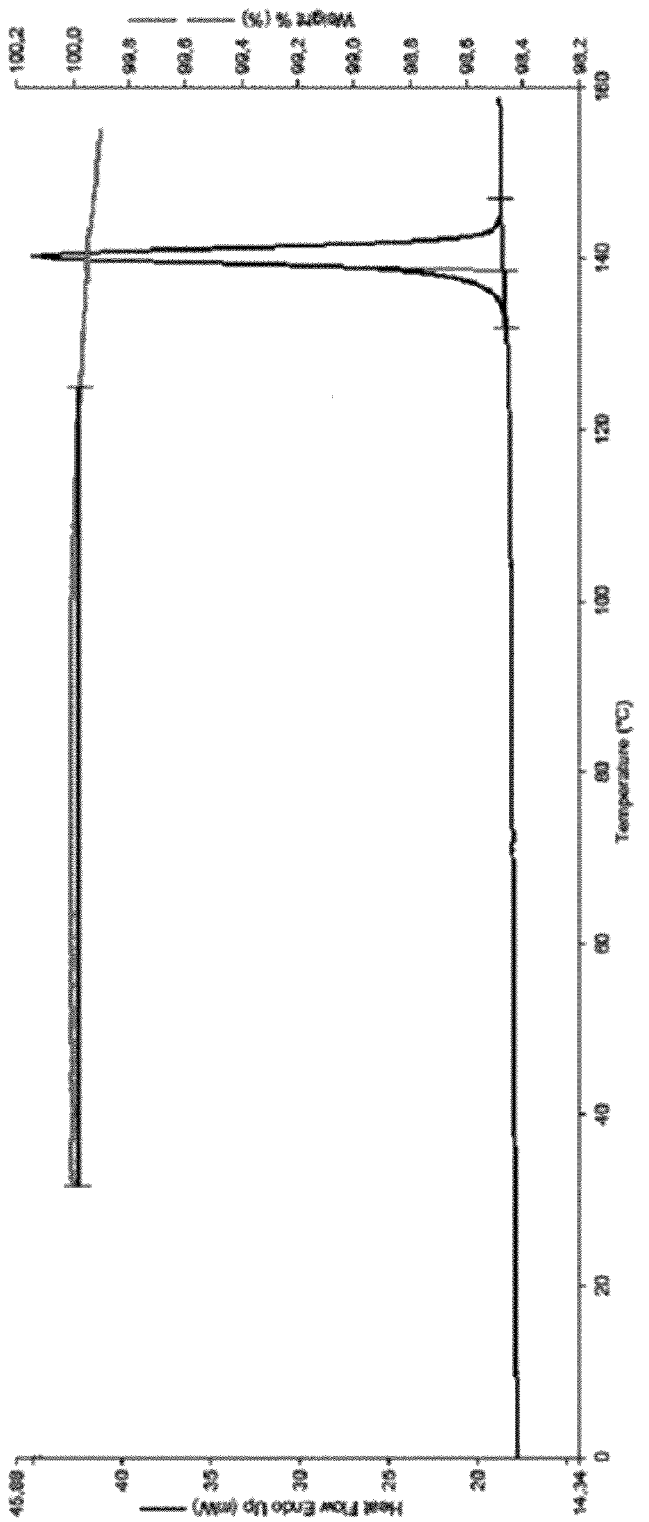


FIG.18

FIG 19A: Schulze Ring Shear Tester

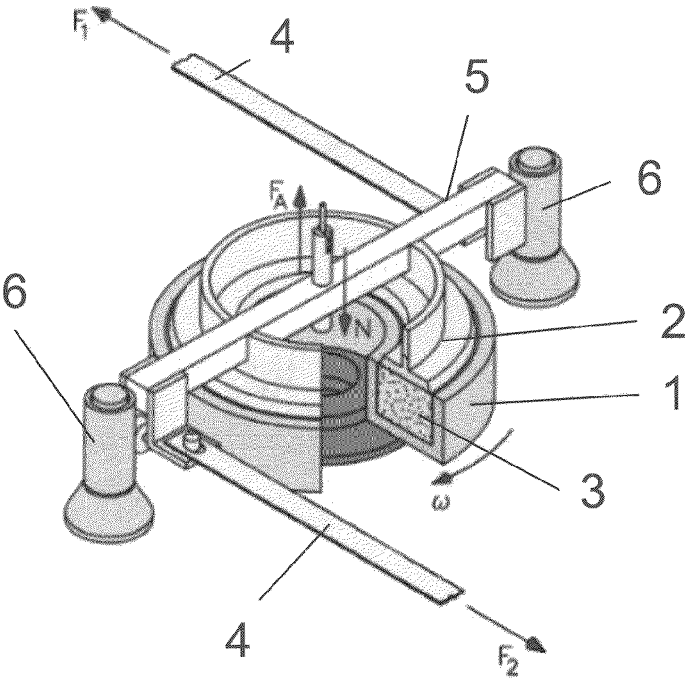


FIG.19A

FIG. 19B: Shear Cell XS-SV3

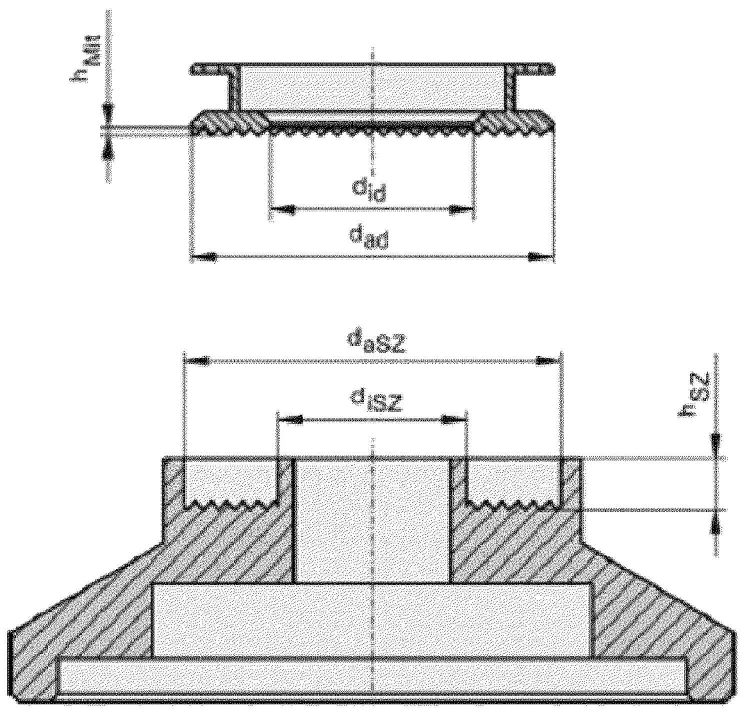


FIG 19B

FIG 20: Result of the Schulze Ring Shear Test

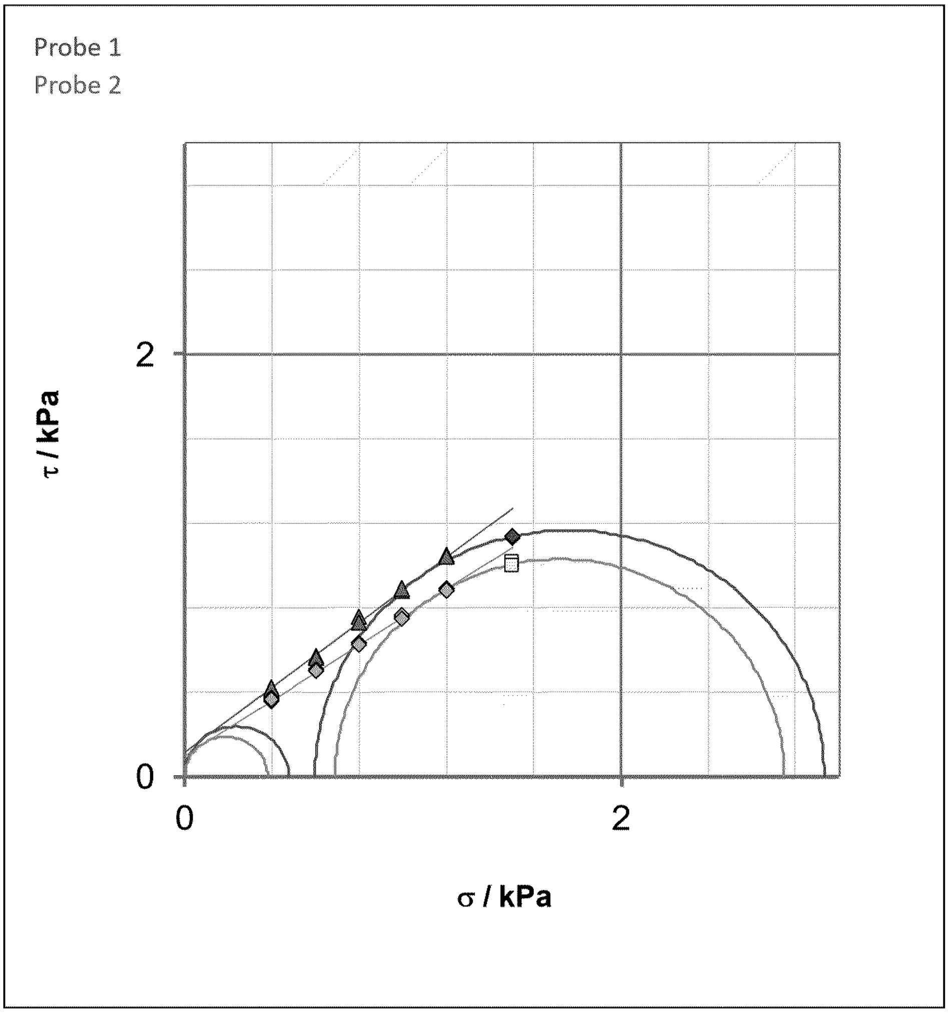


FIG.20

FIG 21: Flow Factor

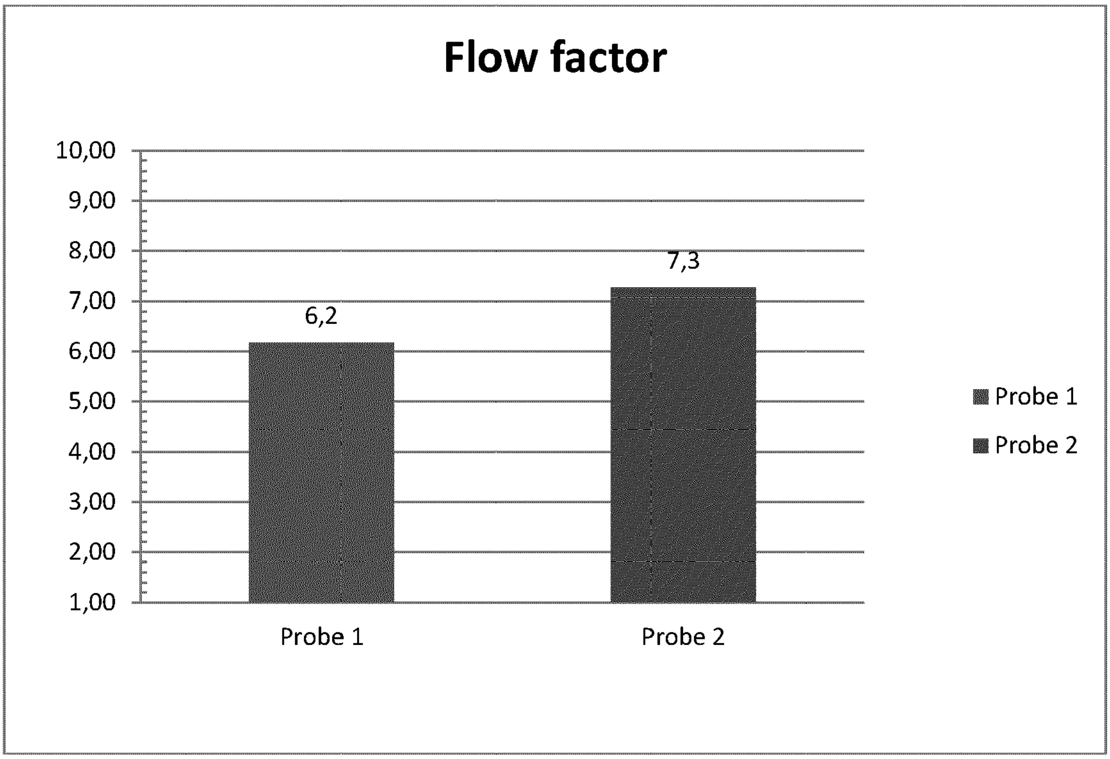


FIG.21

FIG 22: Bulk density

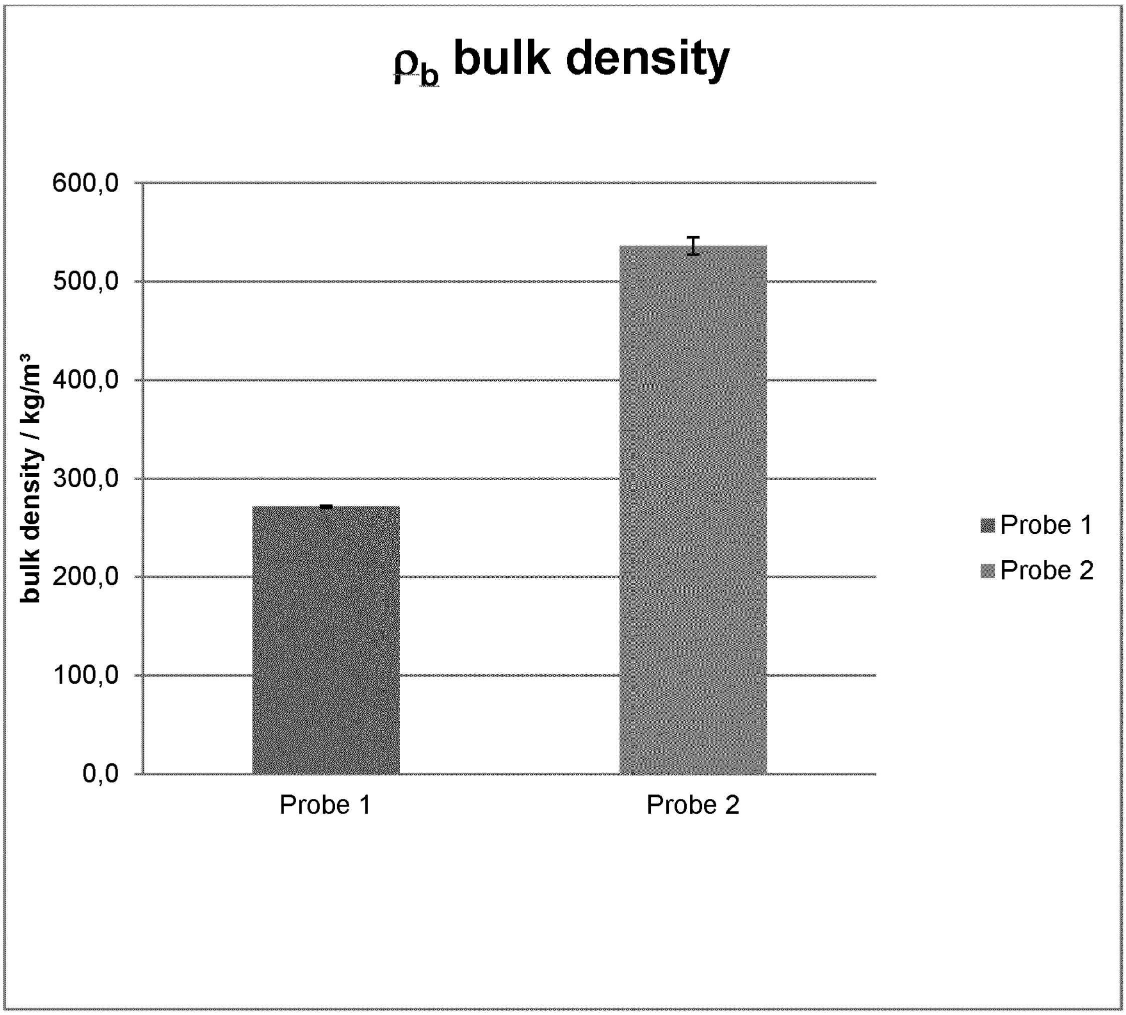


FIG.22

FIG 23: Comparison of particle size distribution

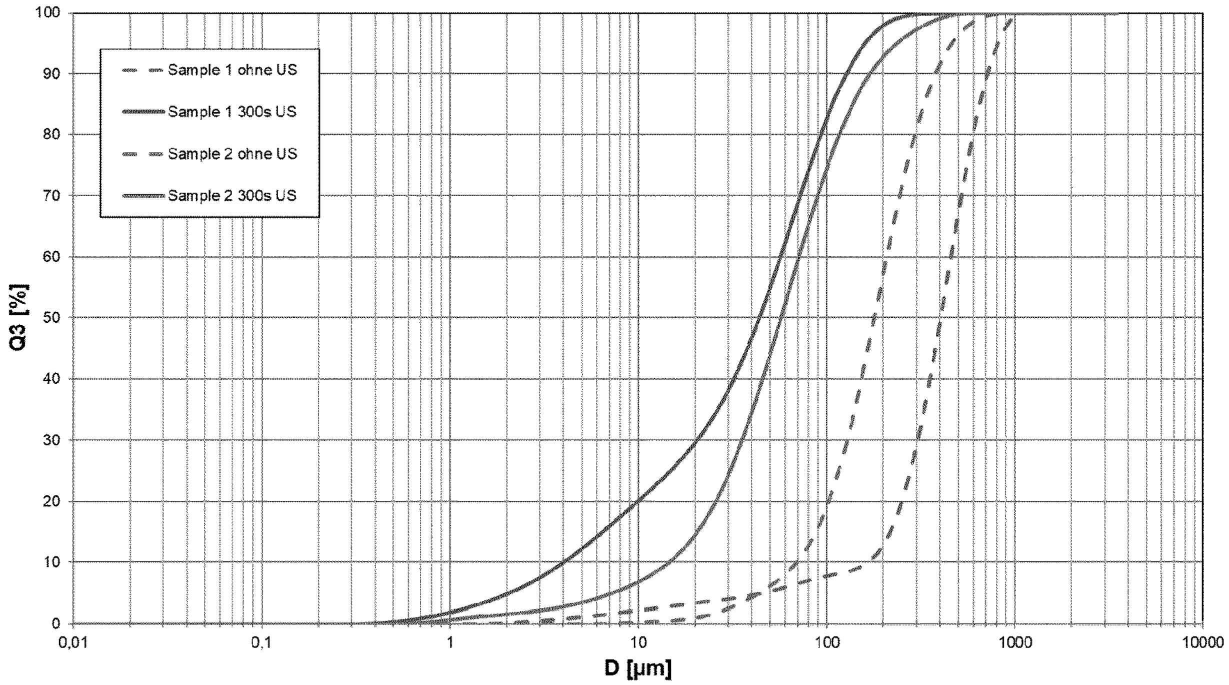


FIG.23

FIG 24A:

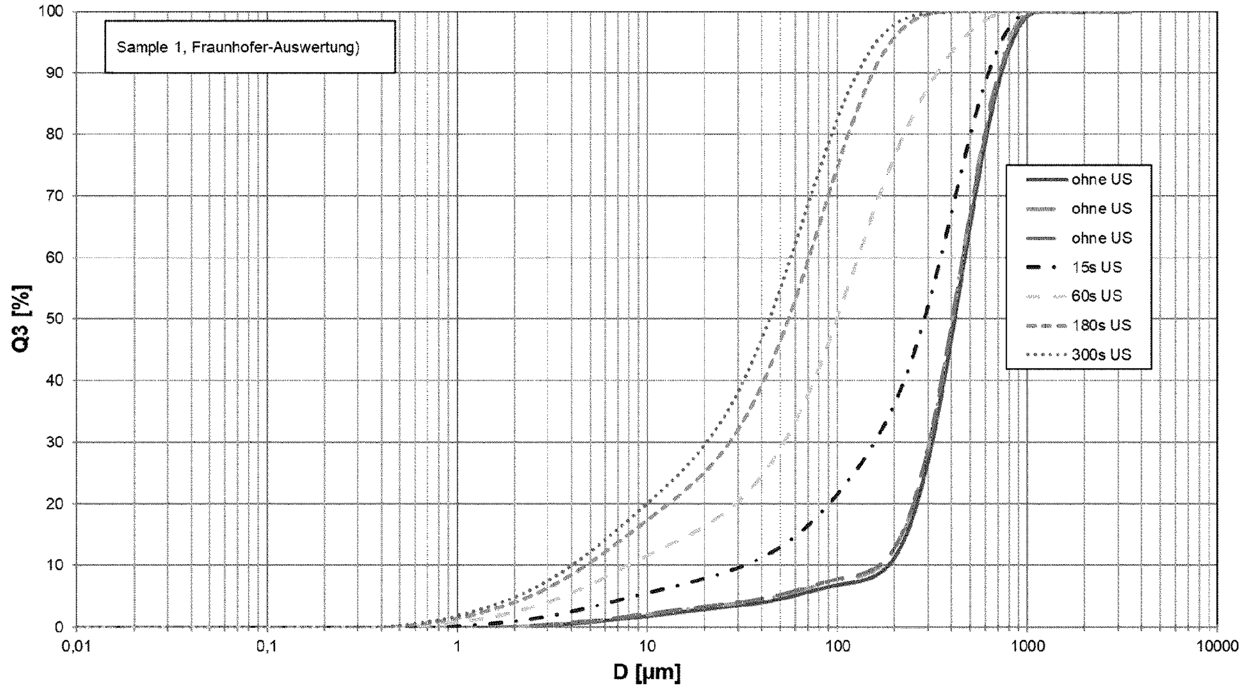


FIG.24A

FIG 24B:

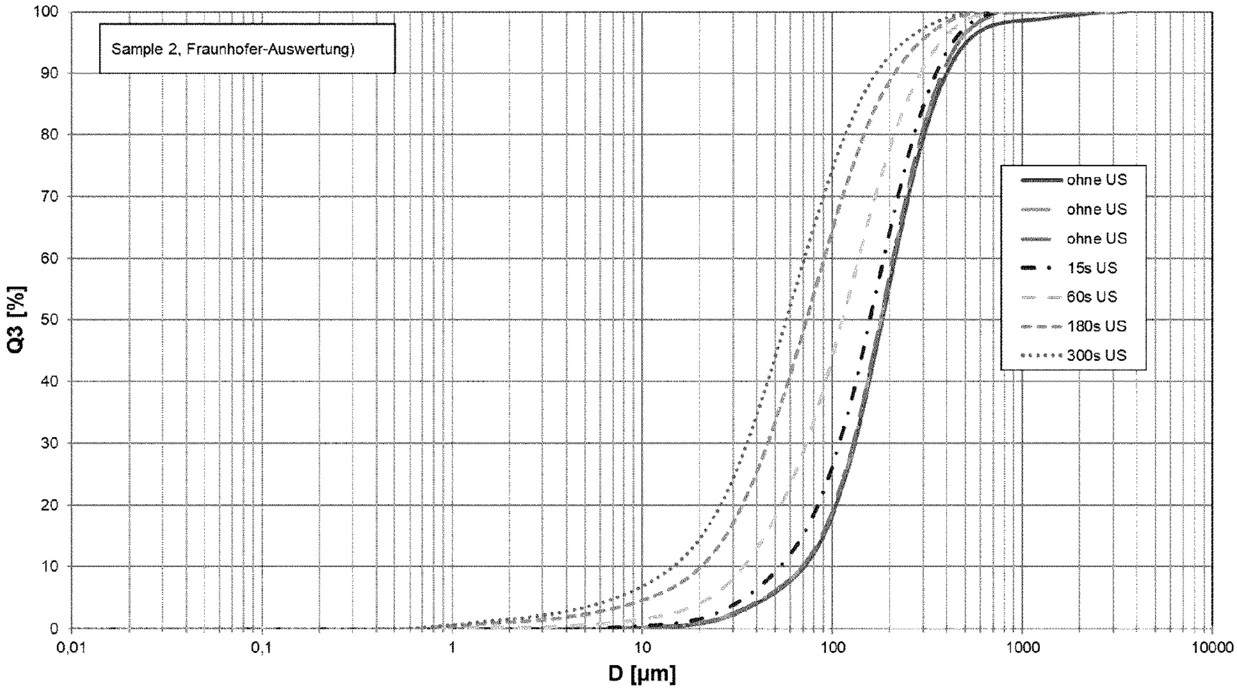


FIG.24B

Europäisches Patentamt
European Patent Office
Office européen des brevets

# EUROPEAN SEARCH REPORT

Application Number
EP 22 17 8275

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br>A | WO 2019/025562 A1 (BAYER AG [DE]; BAYER PHARMA AG [DE] ET AL.) 7 February 2019 (2019-02-07)<br>* claims 1-22; example 135 *<br>----- | 10-14, 19-21<br>1-9, 15-18, 22-25 | INV. C07D273/04 |
| | | | TECHNICAL FIELDS SEARCHED (IPC) |
| | | | C07D |

The present search report has been drawn up for all claims

1

| Place of search | Date of completion of the search | Examiner |
|---|---|---|
| Munich | 14 November 2022 | Sáez Díaz, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 17 8275

This annex lists the patent family members relating to the patent documents cited in the above-mentioned European search report. The members are as contained in the European Patent Office EDP file on
The European Patent Office is in no way liable for these particulars which are merely given for the purpose of information.

14-11-2022

| Patent document cited in search report | Publication date | Patent family member(s) | Publication date |
|---|---|---|---|
| WO 2019025562 A1 | 07-02-2019 | AR 112402 A1 | 23-10-2019 |
| | | AU 2018309356 A1 | 30-01-2020 |
| | | BR 112020002351 A2 | 08-09-2020 |
| | | CA 3071800 A1 | 07-02-2019 |
| | | CL 2020000303 A1 | 13-11-2020 |
| | | CN 111212647 A | 29-05-2020 |
| | | CO 2020001242 A2 | 18-02-2020 |
| | | CR 20200057 A | 16-06-2020 |
| | | CU 20200008 A7 | 30-11-2020 |
| | | EA 202090448 A1 | 21-05-2020 |
| | | EC SP20008441 A | 29-05-2020 |
| | | EP 3661517 A1 | 10-06-2020 |
| | | IL 272098 A | 31-03-2020 |
| | | JP 2020529439 A | 08-10-2020 |
| | | KR 20200036914 A | 07-04-2020 |
| | | MA 49750 A | 10-06-2020 |
| | | PH 12020500134 A1 | 25-01-2021 |
| | | SG 11201913775U A | 30-01-2020 |
| | | TW 201917126 A | 01-05-2019 |
| | | UA 125731 C2 | 25-05-2022 |
| | | US 2020369633 A1 | 26-11-2020 |
| | | UY 37832 A | 28-02-2019 |
| | | WO 2019025562 A1 | 07-02-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019025562 A


### Non-patent literature cited in the description


- **J. PESTI et al.** *Org. Process Res. Dev.,* 2009, vol. 13, 716-728
- Powders and bulk solids. **SCHULZE, D.** Behaviour, Characterization, Storage and Flow. Springer, 2008